(19) Europäisches Patentamt
European Patent Office
Office européen des brevets

(11) Veröffentlichungsnummer: **0 185 359 B1**

# (12) EUROPÄISCHE PATENTSCHRIFT

(45) Veröffentlichungstag der Patentschrift: **04.12.91**

(51) Int. Cl.⁵: **C07D 305/12**, C07D 309/30, C07D 407/12, A61K 31/335

(21) Anmeldenummer: **85116096.0**

(22) Anmeldetag: **17.12.85**

Die Akte enthält technische Angaben, die nach dem Eingang der Anmeldung eingereicht wurden und die nicht in dieser Patentschrift enthalten sind.

## (54) Oxetanone.

(30) Priorität: **21.12.84 CH 6102/84**
**12.09.85 CH 3934/85**

(43) Veröffentlichungstag der Anmeldung:
**25.06.86 Patentblatt 86/26**

(45) Bekanntmachung des Hinweises auf die Patenterteilung:
**04.12.91 Patentblatt 91/49**

(84) Benannte Vertragsstaaten:
**AT BE CH DE FR GB IT LI LU NL SE**

(56) Entgegenhaltungen:
**EP-A- 0 129 748**
**FR-A- 2 379 531**
**FR-A- 2 426 679**

**PATENT ABSTRACTS OF JAPAN, Band 4, Nr. 135, (C-25)(617), 20. September 1980**

(73) Patentinhaber: **F. HOFFMANN-LA ROCHE AG**
**Postfach 3255**
**CH-4002 Basel(CH)**

(72) Erfinder: **Barbier, Pierre, Dr.**
**Rue de Lattre de Tassigny 41**
**F-68170 Rixheim(FR)**
Erfinder: **Schneider, Fernand, Dr.**
**Marignanostrasse 28**
**CH-4059 Basel(CH)**
Erfinder: **Widmer, Ulrich, Dr.**
**Alleeweg 13**
**CH-4310 Rheinfelden(CH)**

(74) Vertreter: **Lederer, Franz, Dr.**
**Lederer, Keller & Riederer Patentanwälte**
**Lucile-Grahn-Strasse 22**
**W-8000 München 80(DE)**

EP 0 185 359 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus Patent Abstracts of Japan 4 (C25)(617)(1980), FR-A-2 426 671, FR-A-2 379 531 und EP-A-0 129 748 sind Esterasehemmer bekannt, die den nachfolgend beschriebenen Oxetanonen der Formeln I und III strukturell teils nah verwandt sind. Die vorliegende Erfindung betrifft neue Oxetanone, Verfahren zu ihrer Herstellung, neue in diesem Verfahren verwendbare Zwischenprodukte, sowie Arzneimittel auf der Basis der besagten Oxetanone oder auf der Basis von Vorläufern davon.

Diese Oxetanone sind Verbindungen der Formel

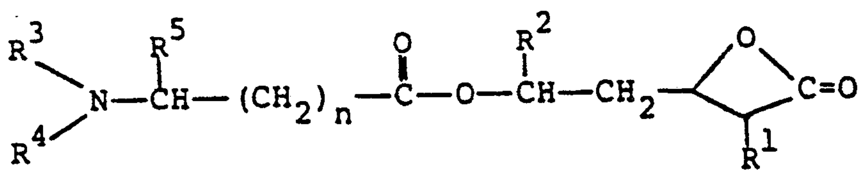

I

worin

| | |
|---|---|
| $R^1$ und $R^2$ | gegebenenfalls durch bis zu 8 Doppel- oder Dreifachbindungen und gegebenenfalls durch ein O- oder S-Atom, das in einer anderen als der α-Stellung zu einem ungesättigten C-Atom vorliegt, unterbrochenes $C_{1-17}$-Alkyl; oder durch 0 bis 3 $C_{1-6}$-Alkyl-(O oder S)$_{1\ oder\ 0}$ ringsubstituiertes Phenyl, Benzyl oder $-C_6H_4-X-C_6H_5$, |
| X | Sauerstoff, Schwefel oder $(CH_2)_{0-3}$ |
| $R^3$ | Wasserstoff, $C_{1-3}$-Alkyl oder $C_{1-3}$-Alkanoyl, |
| $R^4$ | Wasserstoff oder $C_{1-3}$-Alkyl, und |
| $R^5$ | Wasserstoff, eine Gruppe Ar oder Ar-$C_{1-3}$-Alkyl oder gegebenenfalls durch Y unterbrochenes und gegebenenfalls durch Z substituiertes $C_{1-7}$-Alkyl sind, oder |
| $R^4$ mit $R^5$ | einen 4- bis 6-gliedrigen gesättigten Ring bildet, |
| Y | Sauerstoff, Schwefel oder eine Gruppe $N(R^6)$, $C(O)N(R^6)$ oder $N(R^6)C(O)$, |
| Z | eine Gruppe -(O oder S)-$R^7$, $-N(R^7,R^8)$, $-C(O)N(R^7,R^8)$ oder $-N(R^7)C(O)R^8$, |
| n | die Zahl 1 oder 0 ist, wobei falls n die Zahl 1 ist, $R^5$ Wasserstoff ist, |
| Ar | durch 0 bis 3 Gruppen $R^9$ oder $OR^9$ substituiertes Phenyl, und |

$R^6$ bis $R^9$ Wasserstoff oder $C_{1-3}$-Alkyl sind, wobei, falls $R^3$ Formyl und $R^5$ Isobutyl oder $R^3$ Acetyl und $R^5$ Carbamoylmethyl ist, und gleichzeitig $R^2$ Undecyl oder 2,5-Undecadienyl und $R^1$ n-Hexyl ist, $R^4$ eine andere Bedeutung als Wasserstoff hat,

und Salze dieser Oxetanone mit schwachen Säuren.

Die Oxetanone der Formel I bilden mit schwachen Säuren Salze, die ebenfalls Gegenstand der Erfindung sind. Beispiele solcher Säuren sind p-Toluolsulfonsäure, Methansulfonsäure, Oxalsäure, Ascorbinsäure, Fumarsäure, Maleinsäure, Aepfelsäure, Citronensäure und Phosphorsäure.

Die Oxetanone der Formel I können dadurch hergestellt werden, dass man

a) eine Säure der Formel

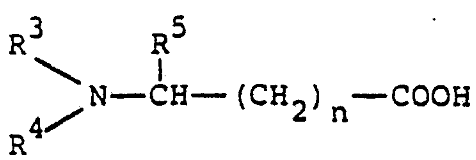

II

oder ein funktionelles Derivat davon mit einem Alkohol der Formel

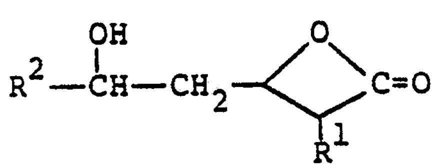

III

worin $R^1$-$R^5$ und n die obige Bedeutung haben, verestert,

b) die Aminoschutzgruppe W in einem Oxetanon der Formel

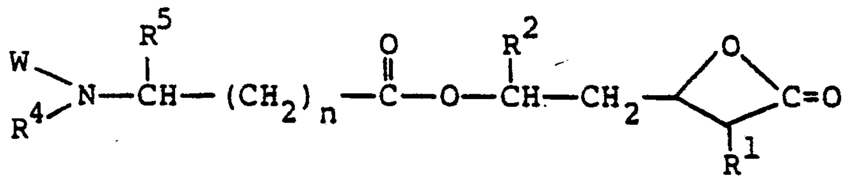

I'

worin $R^1$, $R^2$,$R^4$,$R^5$ und n die obige Bedeutung haben, abspaltet,

c) ungesättigte Reste $R^1$ und $R^2$ gewünschtenfalls katalytisch hydriert,

d) erhaltene Oxetanone der Formel I, worin zumindest eines von $R^3$ und $R^4$ Wasserstoff ist und eine allenfalls in $R^5$ enthaltene Aminogruppe Y oder Z tertiär ist, gewünschtenfalls $C_{1-3}$-alkanoyliert, und

e) erhaltene Oxetanone der Formel I gewünschtenfalls in Form ihrer Salze mit schwachen Säuren isoliert.

Die Oxetanone der Formel I enthalten zumindest 3 asymmetrische C-Atome und die Oxetanone der Formel III können ein oder mehrere asymmetrische C-Atome enthalten. Sie können somit als optisch aktive Enantiomere, als Diastereomere oder als Gemische, z.B. als racemische Gemische, vorliegen.

Die Veresterung a) kann man in einem Lösungsmittel, z.B. einem Aether, wie Tetrahydrofuran (THF), in Gegenwart von Triphenylphosphin und Azodicarbonsäurediäthylester, vorzugsweise bei etwa Raumtemperatur durchführen. Als funktionelles Derivat einer Säure der Formel II kann man das entsprechende Anhydrid verwenden.

Als Beispiel einer Aminoschutzgruppe W in einem Ausgangsoxetanon I' kann man Benzyloxycarbonyl und p-Nitrobenzyloxycarbonyl nennen. Die Abspaltungsreaktion b) kann man durch Hydrierung in einem Lösungsmittel, z.B. einem Aether, wie THF, in Gegenwart eines Hydrierungskatalysators, wie Palladium auf Kohle (Pd/C), vorzugsweise bei Raumtemperatur durchführen.

Die fakultative Hydrierung c) kann man unter ähnlichen Bedingungen wie die oben beschriebene Abspaltungsreaktion b) durchführen.

Die fakultative $C_{1-3}$-Alkanoylierung d) kann in Gegenwart eines Säureanhydrids, z.B. eines gemischten Säureanhydrids, wie Ameisensäureessigsäureanhydrid, in einem Lösungsmittel, z.B. einem Aether, wie THF, vorzugsweise bei Raumtemperatur bewerkstelligt werden.

Die Alkohole III kann man dadurch herstellen, dass man die Aetherschutzgruppe L in einem Aether der Formel

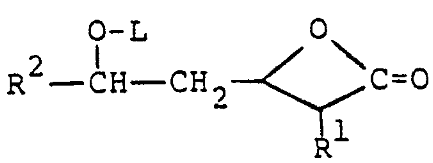

IV

worin $R^1$ und $R^2$ die obige Bedeutung haben, abspaltet.

Beispiele von Aetherschutzgruppen L sind Tetrahydro-2H-pyran-2-yl, 1-Aethoxyäthyl, Benzyl und t-Butyldimethylsilyl.

Die Abspaltung der Aetherschutzgruppe L kann man in einem Lösungsmittel, z.B. einem Alkohol, wie Aethanol, in Gegenwart von Pyridinium-4-toluolsulfonat unter Erhitzen, z.B. auf 50-65 °C durchführen.

Die Aether IV kann man durch Cyclisierung der Säuren der Formel

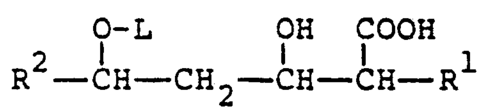

V

herstellen. Diese Reaktion kann man in einem Lösungsmittel, wie Pyridin, unter Abkühlen, z.B. auf 0 °C, in Gegenwart von Benzolsulfochlorid durchführen.

Die Säuren V kann man entweder

a) durch Verseifung entsprechender Ester der Formel

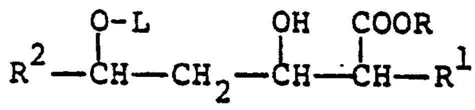

VI

worin R $C_{1-4}$-Alkyl ist und L, $R^1$ und $R^2$ die obige Bedeutung haben, oder
b) durch Kondensation einer Säure der Formel

$R^1$-$CH_2$-COOH VII

mit einem Aldehyd der Formel

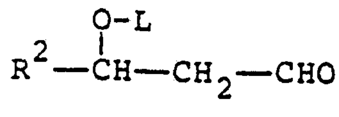

VIII

herstellen.

Beispiele von Alkylresten R sind Methyl, Aethyl und t-Butyl. Die Verseifung a) eines Esters VI kann man mit einer alkoholischen Alkali- bzw. Erdalkalimetallhydroxydlösung, wie einer methanolischen Kaliumhydroxydlösung, durch Erhitzen bei einer Temperatur bis zur Rückflusstemperatur des Reaktionsgemisches durchführen.

Die Kondensation b) der Säure VII mit dem Aldehyd VIII kann man in einem Lösungsmittel, wie THF, in Gegenwart von Diisopropylamin und Butyllithium, unter Abkühlen, z.B. auf -50° C durchführen.

Die Säuren V, die in (5R)- oder (5S)-Form vorliegen, können in folgender Weise in die (2S,3S,5R)- bzw. (2R,3R,5S)-Stereoisomeren übergeführt werden:

Man cyclisiert eine (5R)- oder (5S)-Säure der Formel V, z.B. unter Erhitzen auf 50-60° C in Aethanol mittels Toluol-4-sulfonsäuremonohydrat, zum entsprechenden (6R)- bzw. (6S)-Pyranolon der Formel

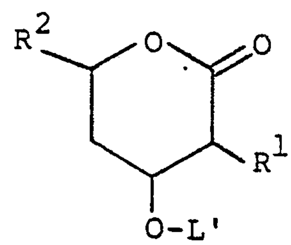

V-A

worin L' für Wasserstoff steht und $R^1$ und $R^2$ die obige Bedeutung haben.
Dieses (6R)- oder (6S)-Pyranolon wird dann, z.B. in Aceton mittels Jones-Reagens bei einer Temperatur unterhalb von 25° C, zum entsprechenden Pyran-2,4-dion oxydiert und letzteres, z.B. in Essigester in Gegenwart von Platinoxyd, stereospezifisch zum (3S,4S,6R)- bzw. (3R,4R,6S)-Pyranolon der Formel V-A, worin L' Wasserstoff ist, hydriert. Dieses Pyranolon wird in eine Verbindung der Formel V-A, worin L' für eine Aetherschutzgruppe, wie t-Butyldimethylsilyl, steht, z.B. in Dimethylformamid mittels t-Butyldimethylchlorsilan, übergeführt. Der erhaltene cyclische (3S,4S,6R)- oder (3R,4R,6S)-Aether wird, z.B. durch Umsetzung mit einer wässrigen Kaliumhydroxydlösung in Dioxan, aufgespalten und die entstandene Verbindung in situ in einen (2S,3S,5R)- bzw. (2R,3R,5S)-Aether der Formel

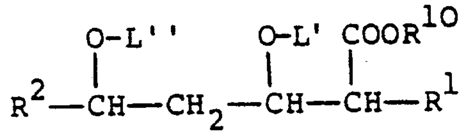

V-B

übergeführt, worin L" für Wasserstoff steht, L' die gleiche Aetherschutzgruppe wie im Aether V-A, $R^{10}$ Benzyl oder p-Nitrobenzyl ist und $R^1$ und $R^2$ die obige Bedeutung haben. Der erhaltene Aether V-B wird dann in einen Diäther der gleichen Formel übergeführt, worin L" für eine Aetherschutzgruppe, wie

Tetrahydro-2H-pyran-2-yl steht. Nach Abspaltung zunächst der Aetherschutzgruppe L', z.B. mit Tetrabutylammoniumfluorid-trihydrat in THF, und dann der Gruppe $R^{10}$, z.B. durch Hydrierung in THF in Gegenwart von Pd/C, erhält man die erwünschte (2S,3S,5R)- bzw. (2R,3R,5S)-Säure der Formel V.

Die Ester VI kann man entweder

a) durch Alkylierung der entsprechenden Ester der Formel

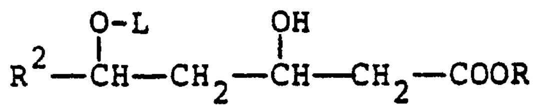

IX

oder

b) durch Reduktion der β-Ketoester der Formel

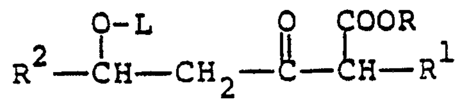

X

herstellen.

Die Alkylierung a) kann man durch Reaktion des Esters IX in einem Lösungsmittel, wie THF, mit einer Lösung von n-Butyllithium in einem Lösungsmittel, wie n-Hexan, in Gegenwart von Diisopropylamin, bei etwa -50 °C, und anschliessende Reaktion mit einer Lösung eines Alkylhalogenids ($R^1$-Hal), z.B. eines Bromids, in Hexamethylphosphorsäuretriamid bei einer Temperatur von etwa 0 bis 10 °C durchführen.

Die Reduktion b) der β-Ketoester X kann man in einem inerten Gas, wie Argon, in einem Lösungsmittel, wie THF, mit einem komplexen Metallhydrid, wie Natriumborhydrid ($NaBH_4$), bei einer Temperatur unterhalb von 0 °C durchführen.

Die Ester IX kann man durch reduktive Entfernung der Sulfoxydgruppe in einem Sulfoxyd der Formel

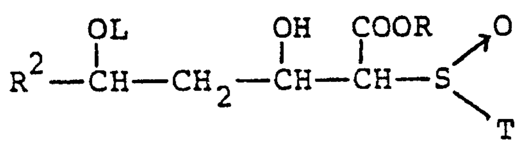

XI

worin T p-Tolyl ist, und L, R und $R^2$ die obige Bedeutung haben,
herstellen. Diese Reaktion kann man z.B. in einem Lösungsmittel, wie THF, mittels Aluminiumamalgam durchführen.

Die β-Ketoester X kann man durch Umsetzung eines Aldehyds der Formel $R^2$-CHO mit einem β-Ketoester der Formel

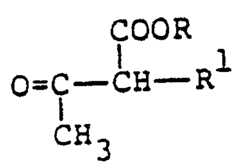

XII

und Verätherung des erhaltenen Alkohole der Formel

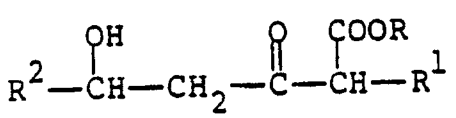

XIII

herstellen.

Die Herstellung des Alkohole XIII bzw. dessen Verätherung kann man wie z.B. in den nachfolgenden

Beispielen H) bzw. J)e) beschrieben durchführen.

In den Zwischenprodukten der Formeln I', III-VI, V-B, X und XIII enthaltene ungesättigte Reste $R^1$ und $R^2$ können gewünschtenfalls hydriert werden, z.B. unter den Bedingungen der weiter oben erwähnten hydrogenolytischen Abspaltung einer Gruppe W oder $R^{10}$.

Die Sulfoxyde XI kann man durch Kondensation eines Aldehyds der obigen Formel VIII mit einem Ester der Formel

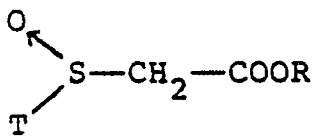

XIV

z.B. wie beschrieben in Beispiel G), herstellen.

Die Aldehyde VIII kann man durch Reduktion der Ester der Formel

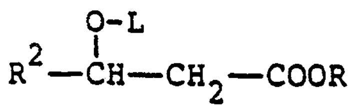

XV

herstellen, z.B. mit einem Di-($C_{1-4}$-alkyl)-aluminiumhydrid, wie Diisobutylaluminiumhydrid, in einem Lösungsmittel, wie Toluol, bei einer Temperatur von etwa -60 bis -80 °C.

Die Ester der Formel XV kann man ausgehend von den Aldehyden der Formel $R^2$-CHO über die Sulfoxyde der Formel

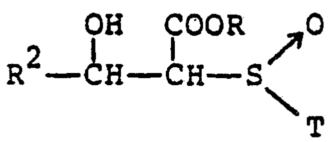

XVI

und die Ester der Formel

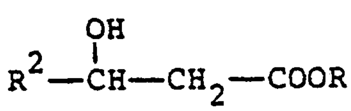

XVII

herstellen, z.B. wie beschrieben in den nachfolgenden Absätzen F)a), d) und f); G)b), d) und f) und J)b), d) und f).

Ferner kann man einen Ester der Formel XV, worin $R^2$ 3-Alkenyl ist, durch Ozonolyse eines Esters der Formel

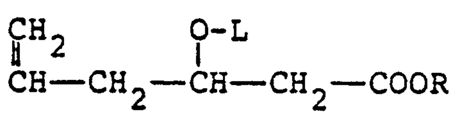

XVIII

und Wittig-Reaktion mit dem erhaltenen Aldehyd der Formel

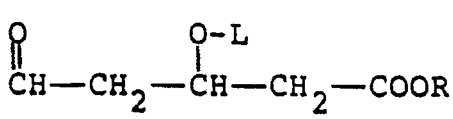

XIX

z.B. wie beschrieben in den Beispielen K) und L) herstellen.

Zur Ueberführung der Aldehyde der Formel VIII bzw. der Formel $R^2$-CHO in die entsprechenden Ester der Formeln IX bzw. XVII kann man an Stelle eines Sulfinylesters XIV das (R)-α-(Hydroxydiphenylmethyl)-benzylacetat verwenden. In diesem Fall erhält man intermediär an Stelle der Sulfoxyde der Formeln XI bzw. XVI die den Alkylestern der Formeln IX bzw. XVII entsprechenden (R)-2-Hydroxy-1,2,2-triphenyläthylester.

Die Oxetanone der Formel I' können in der gleichen Weise wie die Oxetanone der Formel I, z.B. wie beschrieben im nachfolgenden Beispiel 1.10), durch Veresterung einer Säure der Formel II, worin W an Stelle von $R^3$ steht, mit einem Alkohol der Formel III. In dieser Veresterung kann man anstatt der besagten Säure das durch Umsetzung mit N-Aethyl-N'-(3-dimethylaminopropyl)-carbodiimid-hydrochlorid oder vorzugsweise mit Dicyclohexylcarbodiimid erhaltene Säureanhydrid einsetzen, was wie im Beispiel 9 B.1) beschrieben bewerkstelligt werden kann.

Die Herstellung von Zwischenprodukten der Formeln IV bis XIX ist in den folgenden Absätzen A) bis M) näher beschrieben.

A) Herstellung der Aether der Formel IV

A)a) 0,57 g eines Diastereomerengemisches, welches unter anderem aus (2S,3S,5R,13Z,16Z)-2-Hexyl -3-hydroxy-5-[(tetrahydro-2H-pyran -2-yl)oxy]-13,16-docosadiensäure besteht, werden in 10 ml Pyridin gelöst und auf 0°C abgekühlt. Nach tropfenweiser Zugabe von 0,28 ml Benzolsulfochlorid wird längere Zeit bei 0°C gerührt. Die Reaktionsmischung wird auf 120 ml 10-proz. wässrige Kochsalzlösung gegossen und dreimal mit 30 ml Diäthyläther extrahiert. Die vereinigten Extrakte werden getrocknet, filtriert und eingedampft. Nach Chromatographie über Kieselgel erhält man ein Diastereomerengemisch von 3-Hexyl-4-[(10Z,13Z)-2-[(tetrahydro -2H-pyran-2-yl)oxy]-10,13-nonadecadienyl]-2-oxetanonen als farbloses Oel, IR: 1815 $cm^{-1}$.

Auf analoge Art werden erhalten:

A)b) 3-Aethyl-4-[(10Z,13Z) -2-[(tetrahydro-2H-pyran-2-yl)oxy]-10,13-nonadecadienyl] -2-oxetanon, IR: 1820 $cm^{-1}$
aus (13Z,16Z)-2-Aethyl-3-hydroxy-5-[(tetrahydro -2H-pyran-2-yl)oxy]-13,16-docosadiensäure

A)c) (3S,4S)-3-Aethyl-4-[(R,Z)-2-[(tetrahydro -2H-pyran-2-yl)oxy]-10-nonadecenyl-2-oxetanon
aus (2S,3S,5R,Z)-2-Aethyl-3-hydroxy-5-[(tetrahydro -2H-pyran-2-yl)oxy]-13-docosensäure

A)d) (3-Benzyl-4-[(10Z,13Z)-2-[(tetrahydro -2H-pyran-2-yl)oxy]-10,13-nonadecadienyl]-2-oxetanon, IR: 1818 $cm^{-1}$
aus (13Z,16Z)-2-Benzyl-3-hydroxy-5-[(tetrahydro -2H-pyran-2-yl)oxy]-13,16-docosadiensäure

A)e) (3S,4S)-3-Aethyl-4-[(S)-p-phenoxy-β-[(tetrahydro -2H-pyran-2-yl)oxy]phenäthyl]-2-oxetanon
aus (2S,3S,5S)-2-Aethyl-3-hydroxy-5-(p-phenoxyphenyl) -5-[(tetrahydro-2H-pyran-2-yl)oxy]-valeriansäure

A)f) (3S,4S)-3-Hexyl-4[(S)-p-phenoxy-β-[(tetrahydro-2H-pyran-2-yl)oxy]phenäthyl-2-oxetanon, IR: 1815 $cm^{-1}$
aus (2S,3S,5S)-2-Hexyl-3-hydroxy-5-(p-phenoxyphenyl) -5-[(tetrahydro-2H-pyran-2-yl)oxy]-valeriansäure

A)g) 3-Hexyl-4-[2-[(tetrahydro-2H-pyran-2-yl)oxy]tridecyl] -2-oxetanon
aus 2-Hexyl-3-hydroxy-5[(tetrahydro-2H-pyran -2-yl)oxy]hexadecansäure

A)h) 3-Hexyl-4-[(R)-2-[(tetrahydro-2H-pyran -2-yl)oxy]tridecyl]-2-oxetanon
aus 2-Hexyl-3-hydroxy-(R)-5[(tetrahydro-2H-pyran -2-yl)oxy]hexadecansäure

A)i) 3-Aethyl-4-[2-[(tetrahydro-2H-pyran-2-yl)oxy]tridecyl] -2-oxetanon
aus 2-Aethyl-3-hydroxy-5-[(tetrahydro-2H-pyran -2-yl)oxy]hexadecansäure

A)j) 3-Methyl-4-[(R)-2-[(tetrahydro-2H-pyran -2-yl)oxy]tridecyl]-2-oxetanon
aus 2-Methyl-3-hydroxy(R)-5-[(tetrahydro -2H-pyran-2-yl)oxy]hexadecansäure

A)k) 3-Allyl-4-[2-[(tetrahydro-2H-pyran-2-yl)oxy]tridecyl] -2-oxetanon
aus 2-Allyl-3-hydroxy-5-[(tetrahydro-2H-pyran -2-yl)oxy]hexadecansäure

A)l) 3-Hexyl-4-[(R)-2-[(tetrahydro-2H-pyran -2-yl)oxy]propyl]-2-oxetanon
aus 2-Hexyl-3-hydroxy(R)-5[(tetrahydro -2H-pyran-2-yl)oxy]hexansäure

A)m) 3-Hexadecyl-4-[2-[(tetrahydro-2H-pyran -2-yl)oxy]propyl]-2-oxetanon
aus 2-Hexadecyl-3-hydroxy(R)-5-[(tetrahydro -2H-pyran-2-yl)oxy]hexansäure

A)n) 3-Hexyl-4-[(2-[(tetrahydro-2H-pyran -2-yl)oxy]-5-hexenyl]-2-oxetanon
aus 2-Hexyl-3-hydroxy-5-[(tetrahydro -2H-pyran-2-yl)oxy]nonensäure

A)o) 3-Decyl-4-[(R)-2-[(tetrahydro -2H-pyran-2-yl)oxy]-5-hexenyl]-2-oxetanon
aus 2-Decyl-3-hydroxy(R)-5-[(tetrahydro -2H-pyran-2-yl)oxy]nonensäure

A)p) 3-Hexyl-4-[(R)-2-[(tetrahydro-2H-pyran -2-yl)oxy]-5-tri- decenyl-2-oxetanon
aus 2-Hexyl-3-hydroxy(R)-5-[tetrahydro -2H-pyran-2-yl)oxy]hexadecensäure
A)q) 3-Hexyl-4-[(R)-2-[(tetrahydro-2H-pyran -2-yl)oxy]-5-hexenyl]-2-oxetanon
aus 2-Hexyl-3-hydroxy-(R) -5-[(tetrahydro-2H-pyran-2-yl)oxy]nonensäure.

B) Herstellung der Säuren der Formel V

B)a) 1.0 g des rohen Diastereomerengemisches (13Z,16Z)-2-Hexyl-3-hydroxy-5-[(tetrahydro -2H-pyran-2-yl)oxy]-13,16-docosadiensäure-t-butylester wird in 17 ml einer 2N methanolischen Kaliumhydroxidlösung bis zum Verschwinden des Ausgangsmaterials zum Rückfluss erhitzt. Die Reaktionsmischung wird abgekühlt und auf 60 ml Eiswasser gegossen. Durch tropfenweise Zugabe von 1M wässriger Salzsäure wird ein pH von 1 eingestellt und darauf erschöpfend mit Aether extrahiert. Die vereinigten Aetherphasen werden getrocknet, filtriert und eingedampft. Das Oel wird an Kieselgel chromatographiert, wobei ein Diastereomerengemisch von
(13Z,16Z)-2-Hexyl-3-hydroxy-5-[(tetrahydro-2H-pyran -2-yl)oxy]-13,16-docosadiensäure als Oel erhalten wird, IR: 3350, 1709, 1132, 1078, 1023 $cm^{-1}$.
Auf analoge Art werden erhalten:
B)b) (13Z,16Z)-2-Aethyl-3-hydroxy-5-[(tetrahydro-2H-pyran -2-yl)oxy]-13,16-docosadiensäure
aus (13Z,16Z)-2-Aethyl-3-hydroxy -5-[(tetrahydro-2H-pyran-2-yl)oxy]-13,16-docosadiensäure -t-butylester
B)c) (2S,3S,5R,Z)-2-Aethyl-3-hydroxy-5-[(tetrahydro -2H-pyran-2-yl)oxy]-13-docosensäure
aus (2S,3S,5R,Z)-2-Aethyl-3-hydroxy -5-[(tetrahydro-2H-pyran-8-yl)oxy] -13-docosensäure-t-butylester
B)d) (13Z,16Z)-2-Benzyl-3-hydroxy-5-[(tetrahydro -2H-pyran-2- -yl)oxy]-13,16-docosadiensäure, MS: 458 ($M^+$-Dihydropyran); IR: 3008, 1709, 1160, 1134, 1115 $cm^{-1}$
aus (13Z,16Z)-2-Benzyl-3-hydroxy-5-[(tetrahydro -2H-pyran-2-yl)oxy]-13,16-docosadiensäure-t-butylester
B)e) (2S,3S,5S)-2-Aethyl-3-hydroxy-5-(p-phenoxyphenyl) -5-[(tetrahydro-2H-pyran-2-yl)oxy]valeriansäure
aus (2S,3S,5S)-2-Aethyl-3-hydroxy-5-(p-phenoxyphenyl) -5-[(tetrahydro-2H-pyran-2-yl)oxy]-valeriansäure-t-butylester
B)f) (2S,3S,5R)-2-Hexyl-3-hydroxy-5-(p-phenoxyphenyl) -5-[(tetrahydro-2H-pyran-2-yl)oxy]valeriansäure
aus (2S,3S,5R)-2-Hexyl-3-hydroxy-5-(p-phenoxyphenyl) -5-[(tetrahydro-2H-pyran-2-yl)oxy]-valeriansäure-t-butylester
B)g) 2-Hexyl-3-hydroxy-(R)-5-[(tetrahydro-2H-pyran -2-yl)oxy]hexadecansäure
aus 2-Hexyl-3-hydroxy-(R)-5-[(tetrahydro -2H-pyran-2-yl)oxy]hexadecansäure-t-butylester
B)h) 2-Hexyl-3-hydroxy-5-[(tetrahydro-2H-pyran -2-yl)oxy]hexadecansäure
aus 2-Hexyl-3-hydroxy-5-[(tetrahydro-2H-pyran -2-yl)oxy]hexadecansäuremethylester.

C) Herstellung der Säuren V (Variante)

C)a) 2 ml Diisopropylamin in 30 ml trockenem THF werden auf -20° C abgekühlt und darauf 9,68 ml Butyllithium (1,6M/Hexan) zugetropft, sodass die Temperatur -20° C nicht übersteigt. Anschliessend wird 15 Minuten gerührt und dann auf -50° C abgekühlt. Danach werden 0,720 ml 4-Pentensäure in 10 ml THF zugetropft und 10 Minuten weiter bei -50° C gerührt. Man rührt 1 Stunde bei Raumtemperatur und kühlt anschliessend wieder auf -50° C. Nun werden 2 g rac-3-[(Tetrahydro-2H-pyran -2-yl)oxy]-tetradecanol in 10 ml THF zugetropft und man rührt noch 30 Minuten bei -50° C, dann 72 Stunden bei Raumtemperatur. Nach Hydrolyse mit 2N Salzsäure wird das Reaktionsgemisch eingedampft. Der Rückstand wird mit Aether extrahiert. Die organische Phase wird über Natriumsulfat getrocknet, filtriert und eingedampft. Das erhaltene Material wird durch eine Säule von Kieselgel filtriert. Man erhält rohe 2-Allyl-3-hydroxy-5[(tetrahydro-2H-pyran -2-yl)oxy]hexadecansäure.
Auf analoge Weise erhält man:
C)b) 2-Aethyl-3-hydroxy-5-[(tetrahydro-2H-pyran -2-yl)oxy]hexadecansäure
aus rac-3-[(Tetrahydro-2H-pyran-2-yl)oxy]tetradecanal und Butansäure
C)c) 2-Methyl-3-hydroxy(R)-5-[(tetrahydro-2H-pyran -2-yl)oxy]hexadecansäure
aus (R)-3-[(Tetrahydro-2H-pyran-2-yl)oxy]tetradecanal und Propionsäure
C)d) 2-Hexyl-3-hydroxy(R)-5-[(tetrahydro-2H-pyran-2-yl)oxy]hexansäure
aus (R)-3-[(Tetrahydro-2H-pyran-2-yl)oxy]butanal und Octansäure
C)e) 2-Hexadecyl-3-hydroxy-5-[(tetrahydro-2H-pyran -2-yl)oxy]hexansäure

aus 3-[Tetrahydro-2H-pyran-2-yl)oxy]butanal und Octadecansäure

C)f) 2-Hexyl-3-hydroxy-(R)-5[(tetrahydro-2H-pyran-2-yl)oxy]-8-nonensäure
aus (R)-3-[(Tetrahydro-2H-pyran-2-yl)oxy] -6-heptenal und Octansäure

C)g) 2-Decyl-3-hydroxy-(R)-5[(tetrahydro-2H-pyran-2-yl)oxy]-8-nonensäure
aus (R)-3-[(Tetrahydro-2H-pyran -2-yl)oxy]-6-heptenal und Dodecansäure

C)h) 2-Hexyl-3-hydroxy-(R)-5[(tetrahydro-2H-pyran-2-yl)oxy]-8-pentadecensäure
aus (R)-3-[(Tetrahydro-2H-pyran -2-yl)oxy]-6-tetradecenal und Octansäure

C)i) 2-Hexyl-3-hydroxy-5[(tetrahydro-2H-pyran -2-yl)oxy]-8-nonensäure
aus 3-[(Tetrahydro-2H-pyran-2-yl)oxy] -6-heptenal und Octansäure.

D) Herstellung der Ester der Formel VI

D)a) 3,1 ml Diisopropylamin werden unter Argon auf -5° C abgekühlt und 14 ml ca. 1,6M n-Butyllithiumlösung in n-Hexan werden zugetropft. Danach wird 10 Minuten gerührt. Nach Kühlung auf -50° C wird das Kühlbad entfernt und eine Lösung von 5,08 g eines Diastereomerengemisches von (13Z,16Z)-3-Hydroxy-5-[(tetrahydro -2H-pyran-2-yl)oxy]-13,16-docosadiensäure-butylester, in 5 ml THF zugetropft. Die Temperatur steigt dabei auf -20° C. Man lässt auf 0° C aufwärmen und rührt 10 Minuten. Dann wird eine Lösung von 2,1 ml 1-Bromhexan in 2,5 ml Hexamethylphosphorsäuretriamid zugegeben, wobei die Temperatur auf 9° C ansteigt. Danach lässt man auf Raumtemperatur aufwärmen und rührt 2 1/2 Stunden. Die Lösung wird auf 200 ml Eiswasser gegossen und mit Kochsalz gesättigt. Man extrahiert mit Aether. Die vereinigten Extrakte werden getrocknet, filtriert und eingedampft. Das zurückbleibende Oel wird an Kieselgel chromatographiert. Man erhält ein Diastereomerengemisch von (13Z,16Z)-2-Hexyl -3-hydroxy-5-[(tetrahydro-2H-pyran -2-yl)oxy]-13,16-docosadiensäure-t-butylester, MS: 519 ($M^+$-$(CH_3)_3CO$.): IR: 3503, 1728, 1709, 1153.

Auf analoge Art werden erhalten:

D)b) (13Z,16Z)-2-Aethyl-3-hydroxy-5-[(tetrahydro-2H-pyran-2-yl)oxy]-13,16-docosadiensäure-t-butylester, MS: 396 ($M^+$-Dihydropyran-Isobutylen); IR: 3510, 1728, 1153, 1137 $cm^{-1}$
aus (13Z,16Z)-3-Hydroxy-5-[(tetrahydro -2H-pyran-2-yl)oxy]-13,16-docosadiensäure-t-butylester und Aethyljodid

D)c) (13Z,16Z)-2-Benzyl-3-hydroxy -5-[(tetrahydro-2H-pyran-2-yl)oxy]-13,16-docosadiensäure -t-butylester, MS: 525 ($M^+$-$(H_3C)_3$ CO.); IR: 3498, 1725, 1604, 1585, 1496, 1150 $cm^{-1}$
aus (13Z,16Z)-3-Hydroxy-5-[(tetrahydro -2H-pyran-2-yl)oxy]-13,16-docosadiensäure-t-butylester und Benzylbromid

D)d) (2S,3S,5R,Z)-2-Aethyl-3-hydroxy -5-[(tetrahydro-2H-pyran-2-yl)oxy]docosensäure-t-butylester, MS: 465 ($M^+$-$(H_3C)_3CO$.); IR: 3499, 1729, 1155, 1137, 1116 $cm^{-1}$
aus (3S,5R,Z)-3-Hydroxy-5-[(tetrahydro -2H-pyran-2-yl)oxy]-13-docosensäure-t-butylester und Aethyljodid

D)e) (2S,3S,5R)-2-Aethyl-3-hydroxy-5-(p-phenoxyphenyl) -5-[(tetrahydro-2H-pyran-2-yl)oxy]valeriansäure-t-butylester
aus (3S,5R)-3-Hydroxy-5-(p-phenoxyphenyl) -5-[(tetrahydro-2H-pyran-2-yl)oxy]valeriansäure-t-butylester und Aethyljodid

D)f) (2S,3S,5R)-2-Hexyl-3-hydroxy-5-(p-phenoxyphenyl) -5-[(tetrahydro-2H-pyran-2-yl)oxy]valeriansäure-t-butylester,
aus (3S,5R)-3-hydroxy-5-(p-phenoxyphenyl) -5-[(tetrahydro-2H-pyran-2-yl)oxy]valeriansäure-t-butylester und 1-Bromhexan

D)g) 2-Hexyl-3-hydroxy(R)-5-[(tetrahydro-2H-pyran-2-yl)oxy]hexadecansäure-t-butylester, D.C. Kieselgel, Hexan-Diäthyläther 1:1, Rf = 0,65
aus 3-Hydroxy(R)-5-[(tetrahydro-2H-pyran -2-yl)oxy]hexadecansäure-t-butylester und 1-Bromhexan

E) Herstellung der Ester der Formel VI (Variante)

Unter Argonbegasung werden 7,76 g 2-Hexyl-3-oxo-5-[(tetrahydro-2H-pyran -2-yl)oxy]-hexadecansäuremethylester (0,017 Mol) in 500 ml THF gelöst mit 20 ml MeOH versetzt und auf -5° C abgekühlt. Unter Rühren werden 5,3 g Natriumborhydrid (0,14 Mol) portionenweise zugegeben, sodass die Temperatur 0° C nicht übersteigt. Nach 3 Stunden Rühren wird das überschüssige Natriumborhydrid abfiltriert, das Reaktionsgemisch wird mit 2N Salzsäure in der Kälte hydrolysiert (bis pH 6) und das Lösungsmittel wird abgedampft. Der Rückstand wird mit Aether extrahiert und die ätherische Phase getrocknet und abgedampft. Man erhält 7,71 g 2-Hexyl-3-hydroxy-5-[(tetrahydro-2H-pyran -2-yl)oxy]-

hexadecansäure-methylester.

F) Herstellung der Ester der Formeln XVII und IX

F)a) 147,6 g eines Diastereomerengemisches von (11Z,14Z)-3-Hydroxy-2-[(R)-o-tolylsulfinyl] -11,14-eicosadiensäure-t-butylester werden in 5500 ml THF gelöst und dann innerhalb von 6 Stunden mit 190 g amalgamierter Aluminiumfolie versetzt. Dabei wird die Temperatur zwischen 15 und 20° C gehalten. Nach beendeter Zugabe wird solange gerührt, bis die Reaktion beendet ist. Das unlösliche Material wird abgesaugt und zuerst mit 1 l, dann mit 2 l THF gewaschen. Der Filterkuchen wird in 2 l Diäthyläther aufgenommen, verrührt und erneut abgesaugt. Dieses Prozedere wird einmal wiederholt. Die vereinigten organischen Phasen werden eingedampft und der ölige Rückstand wird durch Chromatographie an Kieselgel gereinigt, wobei ein Enantiomerengemisch erhalten wird, welches zu 80% aus

(R,11Z,14Z)-3-Hydroxy-11,14-eicosadiensäure-t-butylester besteht, MS: 324 ($M^+$-Isobutylen); IR: 3452, 1715, 1154 $cm^{-1}$.

Auf analoge Weise werden erhalten:

F)b) (13Z,16Z)-3-Hydroxy-5-[(tetrahydro-2H-pyran-2-yl)oxy] -13,16-docosadiensäure-t-butylester, IR: 3481, 1730, 1153, 1075, 1014 $cm^{-1}$

aus (13Z,16Z)-3-Hydroxy-5-[(tetrahydro-2H-pyran-2-yl)oxy] -2-[(S)-p-tolylsulfinyl]-13,14-docosadiensäure-t-butylester.

F)c) (3S,5R,Z)-3-Hydroxy-5-[(tetrahydro-2H-pyran-2-yl)oxy] -13-docosensäure-t-butylester, MS: 437 ($M^+$-$(H_3C)_3CO$); IR: 3484, 1730, 1655, 1153, 1075, 1024 $cm^{-1}$

aus (3S,5R,Z)-3-Hydroxy-5-[(tetrahydro-2H-pyran-2-yl)oxy] -2-[(S)-p-tolylsulfinyl]-13-docosensäure-t-butylester.

F)d) (R,Z)-3-Hydroxy-11-eicosensäure-t-butylester, IR: 3445, 1716, 1154 $cm^{-1}$

aus (R,Z)-3-Hydroxy-2-[(R)-p-tolylsulfinyl]-11-eicosensäure -t-butylester.

F)e) (3S,5S)-3-Hydroxy-5-(p-phenoxyphenyl) -5-[(tetrahydro-2H-pyran-2-yl)oxy]valeriansäure-t-butylester, MS: 357 ($M^+$-tetrahydropyranyl); IR: 3446, 1727, 1590, 1505, 1489, 1152, 1133, 1118, 1074, 1022 $cm^{-1}$

aus (3S,5S)-3-Hydroxy-5-(p-phenoxyphenyl) -5-[(tetrahydro-2H-pyran-2-yl)oxy]-2-[(S)-o-tolylsulfinyl] valeriansäure-t-butylester.

F)f) [(S)-α-Hydroxy-p-phenoxybenzyl]essigsäure-t-butylester, Smp. 64-65° C (aus n-Hexan), MS: 314 ($M^+$); IR: 3440, 1713, 1590, 1506, 1491, 1158

aus (βS)-β-Hydroxy-p-phenoxy-α-[(R)-p-tolylsulfinyl -hydrozimtsäure-t-butylester.

F)g) 3-Hydroxy-(R)-5-[(tetrahydro-2H-pyran -2-yl)oxy]hexadecansäure-t-butylester

aus 3-Hydroxy-(R)-5-tetrahydro-2H-pyran-2-yl)oxy] -2-[(S)-p-tolylsulfinyl]hexadecansäure-t-butylester.

G) Herstellung der Sulfoxyde der Formeln XI und XVI

G)a) 16,5 g [(S)-p-Tolylsulfinyl]essigsäure-t-butylester werden in ein Gemisch von 600 ml Aether und 60 ml THF gelöst und auf -78° C abgekühlt. Dann werden 43 ml t-Butylmagnesiumbromid zugetropft, sodass die Temperatur unterhalb von -70° C bleibt. Nach 1 Stunde Rühren bei -78° C werden 13,4 g (R)-3-[(Tetrahydro-2H-pyran-2-yl)oxy]-tetradecanal in 100 ml THF zugetropft. Nach 2 Stunden bei -78° C wird das Reaktionsgemisch mit 2N Salzsäure hydrolysiert und das Lösungsmittel wird abgedampft. Die verbleibende Reaktionsmischung wird mit Aether extrahiert und die ätherische Phase wird getrocknet und abgedampft. Nach Chromatographie auf Kieselgel erhält man 14,9 g 3-Hydroxy-(R)-5-[(tetrahydro-2H-pyran-2-yl)oxy] -2-[(S)-p-tolylsulfinyl]-hexadecansäure-t-butylester (67% Ausbeute), Smp. 97-98° C.

In analoger Weise werden erhalten:

G)b) (3R,11Z,14Z)-3-Hydroxy-2-[(R)-p-tolylsulfinyl] -11,14-eicosadiensäure-t-butylester, IR: 3400, 1727, 1653, 1596, 1494, 1279, 1258, 1145, 1085, 1045 $cm^{-1}$

aus 9,12-Octadienal und (R)-p-Tolylsulfinyl-essigsäure-t-butylester.

G)c) (13Z,16Z)-3-Hydroxy-5-[(tetrahydro-2H-pyran-2-yl)oxy] -2-[(S)-p-tolylsulfinyl]-13,16-docosadienylsäure-t-butylester

aus (11Z,14Z)-3-[(tetrahydro-2H-pyran-2-yl)oxy] -11,14-eicosadienal und (S)-p-Tolylsulfinyl-essigsäure-t-butylester.

G)d) (R,Z)-3-Hydroxy-2-[(R)-p-tolylsulfinyl]-11-eicosensäure -t-butylester, MS: 464 ($M^+$-Isobutylen), IR: 3403, 1727, 1596, 1494, 1145, 1043 $cm^{-1}$

aus 9-Octenal und (R)-p-Tolylsulfinyl-essigsäure-t-butylester.

G)e) (3S,5R,Z)-3-Hydroxy-5-[(tetrahydro-2H-pyran-2-yl)oxy] -2-[(S)-p-tolylsulfinyl-13-docosensäure-t-butylester

aus (R,Z)-3-[(tetrahydro-2H-pyran-2-yl)oxy]-11-eicosenal und (S)-p-Tolylsulfinyl-essigsäure-t-butylester.

G)f) (βS)-β-Hydroxy-p-phenoxy-α-[(R)-p-tolylsulfinyl] -hydrozimtsäure-t-butylester, Smp. 126-128 °C (aus n-Hexan)

aus p-Phenoxy-benzaldehyd und (R)-p-Tolylsulfinyl-essigsäure-t-butylester.

G)g) (3S,5S)-3-Hydroxy-5-(p-phenoxyphenyl)-5-[(tetrahydro -2H-pyran-2-yl)oxy]-2-[(S)-p-tolylsulfinyl]-valeriansäure -t-butylester, Smp. 140-145 °C

aus (βS)-p-Phenoxy-β-[(tetrahydro-2H-pyranyl)oxy] -hydrozimtaldehyd und (S)-p-Tolylsulfinyl-essigsäure-t-butylester.

H) Herstellung der Alkohole der Formel XIII

5 g einer 55%-igen Natriumhydriddispersion werden mit Hexan gewaschen und mit 600 ml THF versetzt. Unter Kühlen werden 18,9 g 2-Acetyloctansäure-methylester gelöst in 80 ml THF, zugetropft. Nach 2 Stunden Rühren kühlt man auf -10 °C ab und versetzt unter Kühlung mit 65 ml Butyllithium (1,6M Hexan). Nach 1 Stunde bei -10 °C wird eine Lösung von 19,7 g Dodecanal in 80 ml THF zugetropft. Man lässt auf Raumtemperatur erwärmen und rührt 2 Stunden weiter. Das Reaktionsgemisch wird mit 100 ml 2N Salzsäure hydrolysiert und abgedampft. Der Rückstand wird mit Aether extrahiert und die ätherische Phase wird getrocknet und abgedampft. Nach Chromatographie an Kieselgel erhält man 2-Hexyl-5-hydroxy-3-oxohexadecansäure-methylester, Smp. 38-39 °C.

I) Herstellung der Aldehyde der Formel VIII

I)a) Unter Argonbegasung und Feuchtigkeitsausschluss werden 9,2 g (R)-3-[(Tetrahydro-2H-pyran-2-yl)-oxy] tetradecansäure-t-butylester in 115 ml Toluol gelöst und auf -75 °C abgekühlt. Es werden dann 26,5 ml einer 1,2M Lösung von Diisobutylaluminiumhydrid in Toluol zugetropft, sodass die Temperatur -70 °C nicht übersteigt. Nach 1 Stunde Rühren bei -75 °C werden 7,4 ml gesättigte wässrige Ammoniumchloridlösung und anschliessend 15,5 ml 1N Salzsäure bei -70 °C zugetropft. Dann lässt man auf Raumtemperatur aufwärmen. Nach 1 Stunde Rühren wird die organische Phase getrocknet, filtriert und eingedampft. Das erhaltene Material wird an Kieselgel chromatographiert. Man erhält (R)-3-[(Tetrahydro-2H-pyran-2-yl)oxy]tetradecanal als farbloses Oel.

I)b) rac-3-[(Tetrahydro-2H-pyran-2-yl)oxy]tetradecanal

aus rac-3-[(Tetrahydro-2H-pyran-2-yl)oxy] tetradecansäuremethylester

I)c) (11Z,14Z)-3-[(Tetrahydro-2H-pyran-2-yl)oxy] -11,14-eicosadienal, MS: 291 ($M^+$-2-Tetrahydropyranyloxy), 290 ($M^+$-Tetrahydro-2-pyranol), IR: 2729, 1726, 1132, 1118, 1077 $cm^{-1}$

aus (11Z,14Z)-3-[(Tetrahydro-2H-pyran-2-yl)oxy] -11,14-eicosadiensäure-t-butylester

I)d) (R,Z)-3-[(Tetrahydro-2H-pyran-2-yl)oxy]-11-eicosanal, MS: 292 ($M^+$-Tetrahydro-2-pyranol); IR: 2722, 1726, 1132, 1118, 1077 $cm^{-1}$

aus (R,Z)-3-[(Tetrahydro-2H-pyran-2-yl)oxy] -11-eicosansäure-t-butylester

I)e) (βS)-p-Phenoxy-β-[(tetrahydro-2H-pyran-2-yl)oxy] hydrozimtaldehyd

aus [(S)-p-Phenoxy-α-[(tetrahydro-2H-pyran-2-yl)oxy] benzyl]essigsäure-t-butylester

I)f) (R)-3-[(Tetrahydro-2H-pyran-2-yl)oxy]-6Z-tetradecenal

aus (R)-3-[(Tetrahydro-2H-pyran-2-yl)oxy] -6H-tetradecensäureäthylester.

J) Herstellung der Ester der Formel XV

J)a) 66,5 g (R,11Z,14Z)-3-Hydroxy-11,14-eicosadiensäure -t-butylester, welcher etwa 20% des (S)-Isomeren enthält, und 32 ml frisch destilliertes 3,4-Dihydro-2H-pyran werden in 650 ml Methylenchlorid gelöst und auf 3 °C abgekühlt. Danach werden 640 mg p-Toluolsulfonsäuremonohydrat zugegeben, wobei die Temperatur auf 8 °C ansteigt. Es wird gerührt, bis die Reaktion beendet ist. Darauf wird die Lösung mit einem Gemisch aus 250 ml gesättigter wässriger Kochsalzlösung, 250 ml gesättigter wässriger Natriumhydrogencarbonatlösung und 500 ml Wasser gewaschen. Nach Trocknung wird filtriert und das Lösungsmittel wird entfernt. Der ölige Rückstand wird durch Chromatographie an Kieselgel gereinigt. Man erhält ein Diastereomerengemisch von (11Z,14Z)-3-[(Tetrahydro-2H-pyran-2-yl)oxy] -11,14-eicosadiensäure-t-butylester, MS: 324 ($M^+$-Dihydropyran-Isobutylen); IR: 1731, 1158, 1024 $cm^{-1}$.

In analoger Weise werden erhalten:

J)b) (R,Z)-3-[(Tetrahydro-2H-pyran-2-yl)oxy] -11-eicosensäure-t-butylester, MS: 326 ($M^+$-Dihydropyran-Isobutylen), IR: 1731, 1158, 1134, 1118 $cm^{-1}$

aus (R,Z)-3-Hydroxy-11-eicosensäure-t-butylester und Dihydropyran

J)c) [(S)-p-Phenoxy-α-[(tetrahydro-2H-pyran-2-yl)oxy] benzyl]essigsäure-t-butylester, MS: 313 ($M^+$-Tetrahydropyranyl);IR: 1730, 1590, 1506, 1489, 1391, 1367, 1201, 1149, 1118 $cm^{-1}$

J)d) rac-3-[(Tetrahydro-2H-pyran-2-yl)oxy]tetradecansäure -methylester, D.C. Kieselgel, Hexan-Aether 3:1, Rf = 0,67

aus rac-3-Hydroxytetradecansäuremethylester und Dihydropyran

J)e) 2-Hexyl-3-oxo-5-[(tetrahydro-2H-pyran-2-yl)oxy] hexadecansäure-methylester, Smp. 37-38° C

aus 2-Hexyl-5-hydroxy-3-oxo-hexadecansäure-methylester und Dihydropyran.

K) Herstellung eines Esters der Formel XV (Variante)

K)a) Eine Lösung von 0,51 g Diisopropylamin in 20 ml THF wird mit 3,13 ml einer 1,6 molaren Lösung von Butyllithium in Hexan bei 0° C versetzt. Dann kühlt man auf -78° C ab und gibt 2.3 g Heptyltriphenylphosphoniumbromid hinzu und lässt 5 Minuten bei dieser Temperatur. Man tropft anschliessend eine Lösung von 5-Formyl-(R)-3-[(tetrahydro -2H-pyran-2-yl)oxy]pentancarbonsäureäthylester in 10 ml THF hinzu. Man lässt bei Zimmertemperatur über Nacht rühren. Das Reaktionsgemisch versetzt man mit Wasser, extrahiert mit Aether, trocknet und dampft im Vakuum ein. Den Rückstand chromatographiert man mit Toluol-Essigsäureäthylester (9:1) über Kieselgel und erhält 0,5 g (R)-3-[(Tetrahydro-2H-pyran -2-yl)oxy]-6Z-tetradecencarbonsäureäthylester.

K)b) Auf ähnliche Weise erhält man:

(R)-3-[(Tetrahydro-2H-pyran-2-yl)oxy]-6Z-eicosencarbonsäureäthylester.

L) Herstellung eines Aldehyds der Formel XIX

Eine Lösung von 2,56 g (R)-3-[(Tetrahydro-2H-pyran -2-yl)oxy]-6-heptensäuremethylester in 40 ml Essigsäureäthylester wird bei -75° C mit Ozon behandelt. Nach beendeter Reaktion gibt man 0,1 g Pd auf Kohle hinzu und hydriert bei Zimmertemperatur. Nach beendeter Wasserstoffaufnahme filtriert man den Katalysator ab, wäscht mit Essigsäureäthylester und dampft im Vakuum ein. Man erhält den rohen 5-Formyl-(R)-3-[(tetrahydro-2H-pyran -2-yl)oxy]-pentancarbonsäuremethylester.

M) Auftrennung der Säuren der Formel V in ihre Stereoisomere

M)a) 15,4 g eines Diastereomerengemisches von 2-Hexyl-3-hydroxy-(R)-5-[(tetrahydro-2H)-pyran-2-yl)-oxy]hexadecansäure werden in 160 ml Aethanol gelöst und 800 mg Toluol-4-sulfonsäure-monohydrat werden zugesetzt. Das Reaktionsgemisch wird auf 55-60° C erhitzt, bis die Reaktion beendet ist. Das Lösungsmittel wird im Vakuum entfernt und der Rückstand in 160 ml Dichlormethan gelöst. Man rührt 1 Stunde bei Raumtemperatur. Das Reaktionsgemisch wird eingedampft. Das erhaltene Material wird an Kieselgel chromatographiert. Man erhält das Tetrahydro-3-hexyl-4-hydroxy R-6-undecyl-2H-pyran-2-on, Smp. 95-96° C.

M)b) 3 g eines Diastereomerengemisches von Tetrahydro-3-hexyl-4-hydroxy-(R)-6-undecyl-2H-pyran-2-on werden in 300 ml Aceton gelöst. Unter Rühren werden 3 ml Jones-Reagens zugetropft, so dass die Temperatur 25° C nicht übersteigt. Nach 3 Stunden wird das Reaktionsgemisch auf 700 ml $H_2O$ gegossen. Das Lacton fällt aus und wird abfiltriert. Nach Umkristallisation in Aether/n-Hexan erhält man 1,7 g Tetrahydro-3-hexyl-4-oxo-(R)-6-undecyl-2H-pyran-2-on, Smp. 112,5-113,5° C.

M)c) 8 g eines Isomerengemisches von Tetrahydro-3-hexyl-4-oxo-(R)-6-undecyl-2H-pyran-2-on werden in 2 l Essigester gelöst und 3 g $PtO_2$ werden zugesetzt. Dann hydriert man (50 bar) während 12 Stunden. Der Katalysator wird abfiltriert und die Lösung eingedampft. Nach Umkristallisation erhält man 7 g (3S,4S,6R)-Tetrahydro-3-hexyl-4-hydroxy-6-undecyl -2H-pyran-2-on, Smp. 108-109° C.

M)d) 1,5 g (3S,4S,6R)-Tetrahydro-3-hexyl-4-hydroxy-6-undecyl-2H-pyran-2-on werden in 8 ml DMF gelöst. Dann werden 0,85 g t-Butyldimethylchlorsilan in 4 ml DMF zugetropft. Man rührt 48 Stunden. Das Reaktionsgemisch wird in 100 ml Aether gegossen und mit 1N Salzsäure gewaschen. Die organische Phase wird getrocknet, filtriert und eingedampft. Das erhaltene Material wird an Kieselgel chromatographiert. Man erhält 1,26 g (3S,4S,6R)-Tetrahydro-3-hexyl-4-[(t-butyldimethylsilyl)oxy]-6-undecyl-2H-pyran-2-on, MS: 411 ($M^+$-t-Butyl).

M)e) 0,3 g (3S,4S,6R)-Tetrahydro-3-hexyl -4-[(t-butyldimethylsilyl)oxy]-6-undecyl-2H-pyran-2-on werden in einem Gemisch von 12 ml Dioxan und 0,64 ml 1N wässrigem Kaliumhydroxyd gelöst. Man rührt über Nacht. Dann wird das Reaktionsgemisch eingedampft und in 10 ml Hexamethylphosphortriamid gelöst. Es werden 0,35 ml Benzylbromid zugesetzt. Man rührt 2 Tage. Das Reaktionsgemisch wird auf Wasser

gegossen und mit Aether extrahiert. Die Aetherphase wird getrocknet, filtriert und eingedampft. Das Oel wird an Kieselgel chromatographiert. Man erhält 330 mg (2S,3S,5R)-2-Hexyl-3-[(t-butyldimethylsilyl)oxy] -5-hydroxyhexadecansäurebenzylester, MS: 519 ($M^+$-t-Butyl.

M)f) 350 mg (2S,3S,5R)-2-Hexyl-3-[(t-butyldimethylsilyl)oxy] -5-hydroxyhexadecansäurebenzylester und 0,5 ml frisch destilliertes 3,4-Dihydro-2H-pyran werden in 10 ml Methylenchlorid gelöst und auf -15° C abgekühlt. Man gibt einen Kristall p-Toluolsulfonsäuremonohydrat zu. Es wird gerührt bis die Reaktion beendet ist. Darauf wird die Lösung eingedampft und der Rückstand an Kieselgel chromatographiert. Man erhält 330 mg (2S,3S,5R)-2-Hexyl-3-[(t-butyldimethylsilyl)oxy] -5-[(tetrahydro-2H-pyran-2-yl)oxy]-hexadecansäurebenzylester, MS: 603 ($M^+$-t-Butyl).

M)g) 480 mg (2S,3S,5R)-2-Hexyl-3-[(t-butyldimethylsilyl)oxy] -5-[(tetrahydro-2H-pyran-2-yl)oxy]-hexadecansäurebenzylester und 350 mg Tetrabutylammoniumfluorid-trihydrat werden in 8 ml THF gelöst und 12 Stunden gerührt. Nach Eindampfen wird der Rückstand in 50 ml Aether gelöst und mit Wasser gewaschen. Die ätherische Phase wird getrocknet und eingedampft. Das Rohprodukt wird an Kieselgel chromatographiert. Man erhält 240 mg (2S,3S,5R)-2-Hexyl-3-hydroxy -5-[(tetrahydro-2H-pyran-2-yl)oxy]-hexadecansäurebenzylester, MS: 463 $[(M+H)^+$-Dihydro-2H-pyran-2-yl].

M)h) 430 mg (2S,3S,5R)-2-Hexyl-3-hydroxy -5-[(tetrahydro-2H-pyran-2-yl)oxy]-hexadecansäurebenzylester in 10 ml THF werden mit Pd/C 10% versetzt und 3 Stunden hydriert. Den Katalysator filtriert man ab und nach Eindampfen wird das Rohprodukt an Kieselgel chromatographiert. Man erhält (2S,3S,5R)-2-Hexyl-3-hydroxy -5-[(tetrahydro-2H-pyran-2-yl)oxy]hexadecansäure.

Neu sind die Alkohole der Formel III, worin $R^1$ und $R^2$ die obige Bedeutung haben, wobei falls $R^1$ n-Hexyl ist, $R^2$ eine andere Bedeutung als Undecyl oder 2,5-Undecadienyl hat.

Bevorzugte Oxetanone der Formel I und III sind diejenigen, worin $R^1$ Methyl, Propyl, Hexyl, Decyl, Hexadecyl, Allyl, Benzyl oder inbesondere Aethyl; $R^2$ Methyl, Undecyl, 3-Butenyl, 3-Undecenyl, 8,11-Heptadecadienyl, Phenoxyphenyl oder insbesondere Heptadecyl; $R^3$ Acetyl oder insbesondere Formyl: $R^4$ Methyl oder insbesondere Wasserstoff, und $R^5$ Wasserstoff, Methyl, 2-Butyl, Benzyl, Methylthioäthyl oder insbesondere i-Butyl ist, oder $R^4$ zusammen mit $R^5$ einen Pyrrolidinylrest bildet.

Beispiele von solchen Verbindungen sind:

N-Formyl-L-leucin-1-[(trans-3-äthyl-4-oxo -2-oxetanyl)methyl]dodecylester

N-Formyl-L-leucin-1-[(trans-3-allyl-4-oxo -2-oxetanyl)methyl]dodecylester

N-Formyl-(S)-leucin-(S,9Z,12Z)-1-[(2S,3S)-3-äthyl -4-oxo-2-oxetanyl]methyl]-9,12-octadecadienylester

N-Formyl-(S)-leucin-(S,Z)-1-[[(2S,3S)-3-äthyl-4-oxo -2-oxetanyl]methyl]-9-octadecenylester

N-Formyl-(S)-leucin-(R)-α-[[(2S,3S)-3-äthyl-4-oxo-2-oxetanyl]methyl] -p-phenoxybenzylester.

Besonders bevorzugt ist

N-Formyl-(S)-leucin-(S)-1-[[(2S,3S)-3-äthyl-4-oxo-2-oxetanyl]methyl]octadecylester.

Die Oxetanone der Formel I besitzen wertvolle pharmakologische Eigenschaften. Sie hemmen insbesondere die Pankreaslipase und können dementsprechend bei der Bekämpfung oder Verhütung von Obesitas, Hyperlipämie, Atherosklerose und Arteriosklerose verwendet werden.

Die Hemmung der Pankreaslipase durch die Oxetanone der Formel I kann experimentell gezeigt werden, indem man die bei der Spaltung von Triolein durch Schweinepankreaslipase freigesetzte Oelsäure titrimetrisch erfasst. Zu einer Emulsion, welche 1 mM Taurodeoxycholat, 9 mM Taurocholat, 0,1 mM Cholesterin, 1 mM Eilezithin, 15 mg/ml BSA, 2 mM Tris-HCl, 100 mM Natriumchlorid, 1 mM Calciumchlorid und das Substrat Triolein enthält, gibt man die in Aethanol oder Dimethylsulfoxid (10% des Emulsionsvolumens) gelöste Verbindung der Formel I und startet die Reaktion durch Zugabe von 100 $\mu g$ (175 U) Schweinepankreaslipase. Der pH wird während der Reaktion durch Zugabe von Natronlauge bei 8 gehalten. Aus dem während 10 Minuten ermittelten Verbrauch an Natronlauge wird die $IC_{50}$ berechnet. Die $IC_{50}$ ist diejenige Konzentration, bei der die Lipaseaktivität halbmaximal gehemmt wird. Die nachfolgende Tabelle enthält die für die Verbindungen der Formel I ermittelten $IC_{50}$-Werte und Angaben über die akute Toxizität (Toxizität nach einmaliger oraler Verabreichung an Mäusen).

Tabelle

| Testverbindung in: | $IC_{50}$ in µg/ml | Toxizität in mg/kg p.o. |
|---|---|---|
| Beispiel 1, 8)a) | 0,007 | |
| Beispiel 1, 9) | 0,015 | 5000 |
| Beispiel 1, 16) | 0,02 | |
| Beispiel 1, 18)a) | 0,035 | 2000 |
| Beispiel 1, 20)a) | 0,01 | |
| Beispiel 1, 29) | 0,13 | 4000 |
| Beispiel 1, 1) | 0,11 | |
| Beispiel 4 | 0,20 | |
| Beispiel 5, 2) | 1,0 | |
| Beispiel 6 | 15 | |

Die Oxetanone der Formel I können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden.

Zur Herstellung von pharmazeutischen Präparaten können die erfindungsgemässen Produkte mit pharmazeutisch inerten, anorganischen oder organischen Trägern verabreicht werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze, zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend ein Oxetanon der Formel I, ebenfalls Gegenstand der vorliegenden Erfindung, weiterhin auch ein Verfahren zur Herstellung solcher Arzneimittel, welches dadurch gekennzeichnet ist, dass man ein Oxetanon der Formel I und gegebenenfalls einen oder mehrere andere therapeutisch wertvolle Stoffe in eine galenische Darreichungsform bringt. Wie erwähnt, können die Verbindungen der Formel I bei der Bekämpfung oder Verhütung von Krankheiten verwendet werden und zwar insbesondere bei der Bekämpfung oder Verhütung von Obesitas, Hyperlipämien, Atherosklerose und Arteriosklerose. Die Dosierung kann innerhalb weiter Grenzen variieren und ist natürlich in jedem einzelnen Fall den individuellen Gegebenheiten anzupassen. Im allgemeinen dürfte, insbesondere bei oraler Verabreichung eine Tagesdosis von etwa 0,1 mg bis 100 mg/kg Körpergewicht angemessen sein.

Die Oxetanone der Formel I können auch industriell gefertigten Lebensmitteln zugegeben werden, wobei insbesondere Fette, Oele, Butter, Margarine, Schokolade und andere Konfektartikel in Frage kommen. Solche industriell gefertigte Lebensmittel, die etwa 0,1 bis 5 Gew.-% eines Oxetanons der Formel I erhalten können, und deren Herstellung sind ebenfalls Gegenstand der vorliegenden Erfindung.

Die nachfolgenden Beispiele sollen die vorliegende Erfindung näher erläutern, ihren Umfang jedoch in keiner Weise beschränken. Sämtliche Temperaturen sind in Celsiusgraden angegeben.

Beispiel 1

1.1) Zu einer Lösung von 100 mg (3S,4S)-3-Hexyl-4-[(R)-2-hydroxytridecyl] -2-oxetanon, 74 mg Triphenylphosphin und 45 mg N-Formyl glycin in 2 ml THF tropft man unter Rühren 44,3 ml Azodicarbonsäurediäthylester. Nach Rühren über Nacht wird die organische Phase im Vakuum eingedampft und der Rückstand durch Chromatographie an Kieselgel mit Toluol-Essigsäureäthylester (9:1) gereinigt. Man erhält

den N-Formylglycin-(S)-1-(2S,3S)-[(3-hexyl-4-oxo -2-oxetanyl)methyl]dodecylester, $[\alpha]_D^{25}$ = -22° ($CHCl_3$, c = 0,88),

1.2) durch Veresterung von trans-3-Hexyl-4-(2-hydroxytridecyl)-2-oxetanon mit N-Formylglycin

den N-Formylglycin-1-[(trans-3-hexyl-4-oxo -2-oxetanyl)methyl]dodecylester, D.C. Kieselgel, Diäthyläther-Hexan 9:1, Rf = 0,34

1.3) durch Veresterung von rac-3-Hexyl-4-(2-hydroxytridecyl) -2-oxetanon(2R,3S,4S:2S,3R,4R) mit N-Acetyl-L-leucin

den N-Acetyl-L-leucin-1-[(trans-3-hexyl-4-oxo -2-oxetanyl)methyl]dodecylester, D.C. Kieselgel; $CHCl_3$:Hexan:Dioxan 1:3:0,25, Rf = 0,36

1.4) durch Veresterung von (3S,4S)-3-Hexyl-4-[(R)-2-hydroxytridecyl]-2-oxetanon mit N-Formyl-$\beta$-alanin

den N-Formyl-$\beta$-alanin-(S)-1-(2S,3S)-[(3-hexyl-4-oxo -2-oxetanyl)methyl]dodecylester, D.C. Kieselgel; Toluol-Essigsäureäthylester 2:1, Rf = 0,39

1.5) durch Veresterung von trans-3-Hexyl-[(S)-2-hydroxypropyl]-2-oxetanon(3S,4S:3R,4R) mit N-Formyl-L-leucin

den N-Formyl-L-leucin (S)-1-[(3-hexyl-4-oxo-2-oxetanyl)methyl]ester, D.C. Kieselgel, Toluol-Essigsäureäthylester 2:1, Rf = 0,27

1.6) durch Veresterung von 3-Methyl-4-[(R)-2-hydroxytridecyl]-2-oxetanon(3R,4R:3S,4S) mit N-Formyl-L-leucin

den N-Formyl-L-leucin(S)-1-[(3-methyl-4-oxo -2-oxetanyl)methyl]dodecylester, D.C. Kieselgel, Toluol-Essigsäureäthylester 2:1, Rf = 0,34

1.7) durch Veresterung von rac-trans-3-Hexadecyl-4-(2-hydroxypropyl)-2-oxetanon mit N-Formyl-L-leucin

den N-Formyl-L-leucin-1-[(trans-3-hexadecyl-4-oxo -2-oxetanyl)methyl]äthylester, M.S.: 496 ($M^+$); D.C. Kieselgel, Toluol-Essigsäureathylester 2:1, Rf = 0,44

1.8) durch Veresterung von rac-trans-3-Aethyl-4-(2-hydroxytridecyl)-2-oxetanon mit N-Formyl-L-leucin

1.8)a) den N-Formyl-L-leucin-1-[(trans-3-äthyl-4-oxo-2-oxetanyl)methyl]dodecylester, D.C. Kieselgel, Toluol-Essigsäureäthylester 2:1, Rf = 0,62

1.8)b) den N-Formyl-L-leucin-1-[(trans-3-äthyl-4-oxo-2-oxetanyl)methyl]dodecylester, D.C. Kieselgel, Toluol-Essigsäureäthylester 2:1, Rf = 0,55

1.9) durch Veresterung von rac-trans-3-Allyl-4-(2-hydroxytridecyl)-2-oxetanon mit N-Formyl-leucin

den N-Formyl-L-leucin-1-[(trans-3-allyl-4-oxo -2-oxetanyl)methyl]dodecylester, I.R.: 1825, 1739, 1688; D.C. Kieselgel, Toluol-Essigsäureäthylester 2:1, Rf = 0,58

1.10) durch Veresterung von rac-trans-3-Hexyl-4-(2-hydroxytridecyl)-2-oxetanon mit N-Benzylcarbamoyl-leucin

den N-Benzylcarbamoyl-leucin-1-[(trans-3-hexyl-4-oxo -2-oxetanyl)methyl]dodecylester, D.C. Kieselgel, Hexan-Diäthyläther 1:1, Rf = 0,64

1.11) durch Veresterung von (3S,4S)-3-Hexyl-4-[(R,10Z,13Z)-2-hydroxy-10,13-nonadecadienyl] -2-oxetanon mit N-Formyl-(S)-leucin

den N-Formyl-(S)-leucin(S,9Z,12Z)-1-[[(2S,3S)-3-hexyl-4-oxo -2-oxetanyl]methyl]-9,12-octadecadienylester, M.S.: 575 ($M^+$); I.R.: 1824, 1739, 1675 $cm^{-1}$

1.12) durch Veresterung von rac-trans-3-Hexyl-4-[(10Z,13Z)-2-hydroxy-10,13-nonadecadienyl] -2-oxetanon(2R,3R,4R:2S,3S,4S) mit N-Formyl-(S)-leucin

den N-Formyl-(S)-leucin(9Z,12Z)-1-(trans-3-hexyl)-4-oxo -2-oxetanyl)methyl]-9,12-octadecadienylester (2 Diastereomere), M.S.: 575 ($M^+$); I.R.: 1824, 1740, 1687 $cm^{-1}$

1.13) durch Veresterung von cis-3-Hexyl-4-[(10Z,13Z)-2-hydroxy-10,13-nonadecadienyl] -2-oxetanon (Diastereomerengemisch) mit N-Formyl-(S)-leucin

1.13)a) den N-Formyl-(S)-leucin(9Z,12Z)-1-[(cis-3-hexyl-4-oxo-2-oxetanyl)methyl]-9,12-octadienylester (Diastereomerengemisch I), M.S.: 575 ($M^+$); I.R.: 1823, 1739, 1674 $cm^{-1}$ und

1.13)b) den N-Formyl-(S)-leucin(9Z,12Z)-1-[(cis-3-hexyl-4-oxo-2-oxetanyl)methyl]-9,12-octadienylester (Diastereomerengemisch II), M.S.: 372 ($M^+$-N-Formyl-leucin-$CO_2$); I.R.: 1822, 1739, 1684 $cm^{-1}$

1.14 durch Veresterung von (3S,4S)-3-Benzyl-4-[(R,10Z,13Z)-2-hydroxy -10,13-nonadecadienyl)]-2-oxetanon mit N-Formyl-(S)-leucin

den N-Formyl-(S)-leucin(S,9Z,12Z)-1-[[(2S,3S)-3-benzyl-4-oxo -2-oxetanyl]methyl]-9,12-octadecadienylester, M.S.: 581 ($M^+$); I.R.: 1825, 1739, 1683 $cm^{-1}$

1.15) durch Veresterung von rac-trans-3-Benzyl-4-[(10Z,13Z)-2-hydroxy-10,13-nonadecadienyl]-2-oxetanon(2R,3R,4S:2S,3S,4S) mit N-Formyl-(S)-leucin

1.15)a) den N-Formyl-(S)-leucin(9Z,12Z)-1-[(trans-3-benzyl-4-oxo -2-oxetanyl)methyl]-9,12-octadecadienylester (Diastereomer I), M.S.: 581 ($M^+$); I.R.: 1825, 1739, 1676 $cm^{-1}$ und

1.15)b) den N-Formyl-(S)-leucin(9Z,12Z)-1-[(trans-3-benzyl-4-oxo -2-oxetanyl)methyl]-9,12-octadecadienylester (Diastereomer II), M.S.: 581 ($M^+$); I.R.: 1824, 1740, 1687 $cm^{-1}$

1.16) durch Veresterung von trans-3-Aethyl-4-[(10Z,13Z)-2-hydroxy-10,13-nonadecadienyl] -2-oxetanon (Diastereomerengemisch) mit N-Formyl-(S)-leucin

den N-Formyl-(S)-leucin-(S,9Z,12Z)-1-[(2S,3S)-3-äthyl-4-oxo -2-oxetanyl]methyl]-9,12-octadecadienylester, M.S.: 519 ($M^+$); I.R.: 1825, 1739, 1684 $cm^{-1}$

1.17) durch Veresterung von cis-3-Aethyl-4-[(10Z,13Z)-2-hydroxy-10,13-nonadecadienyl] -2-oxetanon mit N-Formyl-(S)-leucin (Enantiomerengemisch B)

den N-Formyl-(S)-leucin(9Z,12Z)-1-[[cis-3-Aethyl-4-oxo -2-oxetanyl]methyl]-9,12-octadecadienylester (Diastereomerengemisch), M.S.: 316 ($M^+$-N-Formyl-leucin-$CO_2$); I.R.: 1825, 1739, 1677 $cm^{-1}$

1.18) durch Veresterung von (3S,4S)-3-Aethyl-4-[(R,Z)-2-hydroxy-10-nonadecenyl]-2-oxetanon mit N-Formyl-S-leucin

1.18)a) den N-Formyl-(S)-leucin(S,Z)-1-[[(2S,3S)-3-äthyl-4-oxo -2-oxetanyl]methyl]-9-octadecenylester (Diastereomer I), M.S.: 521 ($M^+$); I.R.: 1825, 1739, 1673 $cm^{-1}$ und

1.18)b) den N-Formyl-(S)-leucin(Z)-1-[(trans-3-äthyl-4-oxo -2-oxetanyl)methyl]-9-octadecenylester

1.19) durch Veresterung von (3S,4S)-3-Hexyl-4-[(S)-$\beta$-hydroxy-p-phenoxyphenäthyl] -2-oxetanon mit N-Formyl-(S)-leucin

den N-Formyl-(S)-leucin $\alpha$-[[(2S,3S)-3-hexyl-4-oxo-2-oxetanyl]methyl] -p-phenoxybenzylester (Diastereomerengemisch), M.S.: 509 ($M^+$); I.R.: 1821, 1742, 1686 $cm^{-1}$

1.20) durch Veresterung von (3S,4S)-3-Aethyl-4-[(S)-$\beta$-hydroxy-p-phenoxyphenäthyl] -2-oxetanon mit N-Formyl-(S)-leucin

1.20)a) den N-Formyl-(S)-leucin (R)-$\alpha$-[[(2S,3S)-3-äthyl-4-oxo-2-oxetanyl]methyl] -p-phenoxybenzylester, M.S.: 453 ($M^+$); I.R.: 1824, 1742, 1686 $cm^{-1}$ und

1.20)b) den N-Formyl-(S)-leucin(S)-$\alpha$-[[(2S,3S)-3-äthyl-4-oxo -2-oxetanyl]methyl]-p-phenoxybenzylester, M.S.: 453 ($M^+$); I.R.: 1823, 1743, 1686 $cm^{-1}$

1.21) durch Veresterung von rac-trans-3-Hexyl-4-(2-hydroxy-5-hexenyl)-2-oxetanon mit N-Formyl-L-leucin

den N-Formyl-L-leucin-1-[(trans-3-hexyl-4-oxo-2-oxetanyl)methyl] -4-pentenylester (Gemisch von 2 Diastereomeren)

1.22) durch Veresterung von (S)-3-Hexyl-(S)-4-[(R)-2-hydroxy-5-hexenyl)-2-oxetanon mit N-Formyl-L-leucin

den N-Formyl-L-leucin(S)-1-[[(2S,3S)-3-hexyl-4-oxo -2-oxetanyl]methyl]-4-pentenylester

1.23) durch Veresterung von (S)-3-Hexyl-(S)-4-[(B)-2-hydroxy-5-hexenyl)-2-oxetanon mit N-Formyl-(S)-valin

den N-Formyl-(S)-valin-1-[[(2S,3S)-3-hexyl-4-oxo -2-oxetanyl]methyl]-4-pentenylester

1.24) durch Veresterung von (S)-3-Hexyl-(S)-4-[(R)-2-hydroxy-5-hexenyl)-2-oxetanon mit N-Formyl-L-isoleucin

den N-Formyl-L-isoleucin(S)-1-[(2S,3S)-3-hexyl-4-oxo -2-oxetanyl]methyl]-4-pentenylester

1.25) durch Veresterung von (S)-3-Hexyl-(S)-4-[(R)-2-hydroxy-5-hexenyl)-2-oxetanon mit N-Formyl-L-phenylalanin

den N-Formyl-L-phenylalanin(S)-1-[(2S,3S)-3-hexyl-4-oxo -2-oxetanyl)methyl]-4-pentenylester

1.26) durch Veresterung von (S)-3-Hexyl-(S)-4-[(R)-2-hydroxy-5-hexenyl)-2-oxetanon mit N-Formyl-L-alanin

den N-Formyl-L-alanin-(S)-1-[(2S,3S)-3-hexyl-4-oxo -2-oxetanyl)-4-pentenylester

1.27) durch Veresterung von (S)-3-Hexyl-(S)-4-[(R)-2-hydroxy-5-hexenyl)-2-oxetanon mit N-Formyl-L-prolin

den N-Formyl-L-prolin(S)-1-[(2S,3S)-3-hexyl-4-oxo -2-oxetanyl)methyl]-4-pentenylester

1.28) durch Veresterung von (S)-3-Hexyl-(S)-4-[(R,Z)-2-hydroxy-5-tridecenyl)-2-oxetanon mit N-Formyl-L-leucin

den N-Formyl-L-leucin-(S,Z)-1-[[(2S,3S)-3-hexyl-4-oxo -2-oxetanyl]methyl]-4-dodecenylester

1.29) durch Veresterung von (S)-3-Decyl-(S)-4-[(R)-2-hydroxy-5-hexenyl)-2-oxetanon mit N-Formyl-L-leucin

den N-Formyl-L-leucin(S)-1-[[(2S,3S)-3-decyl-4-oxo -2-oxetanyl]methyl]-4-pentenylester

1.30) durch Veresterung von (S)-3-Hexyl-(S)-4-[(R)-2-hydroxy-5-hexenyl]-2-oxetanon mit N-Formyl-L-methionin

den N-Formyl-L-methionin(S)-1-[(2S,3S)-3-hexyl-4-oxo -2-oxetanyl)methyl]-4-pentenylester.

1.31) durch Veresterung von 3-Aethyl-4-[(10Z,13Z)-2-hydroxy -10,13-nonadecadienyl)-2-oxetanon mit N-Formyl-N-methyl-L-leucin
den N-Formyl-N-methyl-L-leucin(9Z,12Z) -1-[(3-äthyl-4-oxo-2-oxetanyl)methyl]-9,12-octadienylester.

Beispiel 2

Einer Lösung von 27 mg N-Formyl-(S)-leucin-(S,9Z,12Z)-1-[(2S,3S)-3-hexyl-4-oxo -2-oxetanyl]methyl]-9,12-octadienylester in 1 ml THF werden 4,4 mg 10% Pd/C zugesetzt. Es wird bis zur Beendigung der Reaktion bei Raumtemperatur hydriert. Der Katalysator wird abfiltriert und das Lösungsmittel im Vakuum entfernt. Nach Trocknung im Vakuum erhält man N-Formyl-(S)-leucin-(S)-1-[[(2S,3S)-3-hexyl-4-oxo -2-oxetanyl]methyl]octadecylester als weisse Kristalle, Smp. 64-65° C.

Beispiel 3

Analog Beispiel 2 erhält man:

3.1) aus N-Formyl-(S)-leucin-(S,9Z,12Z)-1-[(2S,3S)-3-äthyl-4-oxo -2-oxetanyl]methyl]-9,12-octadecadienylester
den N-Formyl-(S)-leucin-(S)-1-[[(2S,3S)-3-äthyl-4-oxo -2-oxetanyl]methyl]octadecylester als weisse Kristalle, Smp. 48-53° C

3.2) aus N-Formyl-L-leucin-1-[(trans-3-allyl-4-oxo -2-oxetanyl]methyl]dodecylester
den N-Formyl-L-leucin-1-[(trans-3-propyl-4-oxo -2-oxetanyl]methyl]dodecylester.

Beispiel 4

Eine Lösung von 10 mg N-Formyl-L-leucin-1-[(trans-3-hexyl-4-oxo -2-oxetanyl)methyl]-4-pentenylester in 0,5 ml THF wird mit 2,5 mg 5% Pd/C versetzt und hydriert. Nach beendeter Wasserstoffaufnahme filtriert man den Katalysator ab und dampft im Vakuum ein. Den Rückstand chromatographiert man mit Toluol-Essigsäureäthylester (8:2) über Kieselgel und erhält amorphes N-Formyl-L-leucin-1-[(trans-3-hexyl-4-oxo -2-oxetanyl]methyl]pentylester als ein Gemisch von 2 Diastereomeren.

Beispiel 5

Analog Beispiel 4 erhält man:

5.1) aus N-Formyl-L-alanin-(S)-1-[[(2S,3S)-3-hexyl-4-oxo -2-oxetanyl]methyl]-4-pentenylester
den N-Formyl-L-alanin-(S)-1-[[(2S,3S)-3-hexyl-4-oxo -2-oxetanyl]methyl]pentylester

5.2) aus N-Formyl-L-phenylalanin (S)-1-[(2S,3S)-3-hexyl-4-oxo-2-oxetanyl]methyl] -4-pentenylester
den N-Formyl-L-phenylalanin (S)-1-[[(2S,3S)-3-hexyl-4-oxo-2-oxetanyl]methyl] -4-pentylester

5.3) aus N-Formyl-L-leucin (S)-1-[[(2S,3S)-3-decyl-4-oxo-2-oxetanyl]methyl] -4-pentenylester
den N-Formyl-L-leucin (S)-1-[[(2S,3S)-3-decyl-4-oxo-2-oxetanyl]methyl]pentylester.

Beispiel 6

Eine Lösung von 67 mg N-Benzylcarbamoyl-leucin-1-[(trans -3-hexyl-4-oxo-2-oxetanyl)-methyldodecylester in 15 ml THF wird in Gegenwart von 10% Pd/C bei Raumtemperatur unter einer $H_2$-Atmosphäre (Normaldruck) bis zum vollständigen Umsatz hydriert. Das nach Filtrieren und Eindampfen erhaltene Produkt wird an Kieselgel chromatographiert. Man erhält reinen Leucin-1-[(trans-3-hexyl-4-oxo -2-oxetanyl)methyl]dodecylester, Smp. 27-30° C.

Beispiel 7

265 mg eines Diastereomerengemisches von 3-Hexyl-4-[(10Z,13Z)-2-[tetrahydro -2H-pyran-2-yl)oxy]-10,13-nonadecadienyl]-2-oxetanon werden in 2,5 ml Aethanol gelöst und 13 mg Pyridinium-4-toluolsulfonat zugesetzt. Das Reaktionsgemisch wird auf 55-60° C erhitzt, bis die Reaktion beendet ist. Das Lösungsmittel wird im Vakuum entfernt und der Rückstand in Aether aufgenommen, wobei Kristalle ausfallen, die durch Filtration entfernt werden. Das Lösungsmittel wird im Vakuum abgedampft und der Rückstand an Kieselgel chromatographiert, wobei die unten aufgeführten Produkte in der angegebenen Reihenfolge eluiert werden. Die teilweise noch verunreinigten Produkte kann man durch Wiederholung der Chromatographie reinigen.

Auf diese Art werden erhalten:

8.1) ((3S,4S)-4-Hexyl-4-[(R,10Z,13Z) -2-hydroxy-10,13-nonadecadienyl]-2-oxetanon (Diastereomer I) als farbloses Oel, MS: $M^+$ (434); IR: 3420, 1820, 1120 $cm^{-1}$

8.2) rac-trans-3-Hexyl-4-[(10Z,13Z) -2-hydroxy-10,13-nonadecadienyl]-2-oxetanon (Diastereomer II) als farbloses Oel, MS: $M^+$ (434); IR: 3448, 1820, 1122 $cm^{-1}$

8.3) cis-3-Hexyl-4-[(10Z,13Z) -2-hydroxy-10,13-nonadecadienyl]-2-oxetanon (Diastereomer III) als farbloses Oel, MS: $M^+$ (434): IR: 3374, 1822, 1117 $cm^{-1}$.

Beispiel 8

Analog Beispiel 7 wurden erhalten:

8.A.1) trans-3-Aethyl-4-[(10Z,13Z) -2-hydroxy-10,13-nonadecadienyl]-2-oxetanon, MS: 360 ($M^+$-$H_2O$), 334 ($M^+$-$CO_2$), 316 ($M^+$-$H_2O$-$W_2$), IR: 3446, 1823, 1122 $cm^{-1}$

8.A. 2) cis-3-Aethyl-4-[(10Z,13Z) -2-hydroxy-10,13-nonadecadienyl]-2-oxetanon (Enantiomerengemisch A), MS: 378/$M^+$); IR: 3445, 1822, 1116 $cm^{-1}$ und

8.A.3) cis-3-Aethyl-4-[(10Z,13Z) -2-hydroxy-10,13-nonadecadienyl]-2-oxetanon (Enantiomerengemisch B), MS: (chemische Induktion mit $NH_3$): 396 (M + $NH_4^+$), 374 (M + $H^+$); IR: 3415, 1823, 1115 $cm^{-1}$

aus einem cis,trans-Gemisch von 3-Aethyl-4-[(R,10Z,13Z) -2-[tetrahydro-2H-pyran-2-yl)oxy]-10,13-nonadecadienyl] -2-oxetanon.

8.B. 3-Aethyl-4-[(Z)-2-hydroxy -10-nonadecenyl]-2-oxetanon, MS: 362 ($M^+$-$H_2O$), 318 ($M^+$-$H_2O$-$10_2$); IR: 3435, 1823, 1119 $cm^{-1}$

aus 3-Aethyl-4-[(Z)-2-[(tetrahydro -2H-pyran-2-yl)oxy]-10-nonadecenyl-2-oxetanon

8.C.1) (3S,4S)-3-Benzyl-4[(R,10Z,13Z) -2-hydroxy-10,13-nonadecadienyl]-2-oxetanon, MS: 440 ($M^+$); IR: 3430, 1822, 1120 $cm^{-1}$

8.C.2) rac-trans-3-Benzyl-4[(10Z,13Z) -2-hydroxy-10,13-nonadecadienyl]-2-oxetanon, MS: 440 ($M^+$); IR: 3512, 1822, 1123 $cm^{-1}$ und

8.C.3) cis-3-Benzyl-4-[(10Z,13Z) -2-hydroxy-10,13-nonadecadienyl]-2-oxetanon (2 Diastereomere), MS: 378 ($M^+$-$CO_2$-$H_2O$), 287 ($M^+$-$H_2O$-$CO_2$-Benzyl); IR: 3420, 1822, 1134 $cm^{-1}$

aus einem Diastereomerengemisch von 3-Benzyl-4-[(R,10Z,13Z) -2-[tetrahydro-2H-pyran-2-yl)oxy]-10,13-nonadecadienyl] -2-oxetanon.

8.D. (3S,4S)-3-Hexyl-4-[(S)-β-hydroxy -p-phenoxyphenäthyl]-2-oxetanon, Smp. 51-54°, MS: 368 ($M^+$); IR: 3486, 1793, 1245, 1141

aus (3S,4S)-3-Hexyl-4-[(S) -p-phenoxy-β-[(tetrahydro-2H-pyran-2-yl)oxy]phenäthyl] -2-oxetanon.

8.E. (3S,4S)-3-Aethyl-4-[(S)-β-hydroxy -p-phenoxyphenäthyl]-2-oxetanon, Smp. 67-70° C, MS: 312 ($M^+$); IR: 3416, 1835, 1250, 1108

aus (3S,4S)-3-Aethyl-4-[(S) -p-phenoxy-β-[(tetrahydro-2H-pyran -2-yl)oxy]phenäthyl]-2-oxetanon.

8.F.1. rac-trans-3-Hexyl-4-(2-hydroxytridecyl) -2-oxetanon(2R,3S,4S:2S,3R,4R), Smp. 44,5-46°,

8.F.2. rac-trans-3-Hexyl-4-(2-hydroxytridecyl) -2-oxetanon(2S,3S,4S:2R,3R,4R), Smp. 45,5-47° C,

8.F.3. rac-cis-3-Hexyl-4-(2-hydroxytridecyl) -2-oxetanon (Enantiomerenpaar A) D.C. Kieselgel, Hexan-Essigsäureäthylester 9:1, Rf = 0,49 und

8.F.4. rac-cis-3-Hexyl-4-(2-hydroxytridecyl) -2-oxetanon (Enantiomerenpaar B), D.C. Kieselgel, Hexan-Essigsäureäthylester 9:1, Rf = 0,46

aus 3-Hexyl-4-[2-[(tetrahydro -2H-pyran-2-yl)oxy]tridecyl]-2-oxetanon.

8.G.1. (3S,4S)-3-Hexyl-4-[(R)-2-hydroxytridecyl] -2-oxetanon, Smp. 46-46,5° C und

8.G.2. (3R,4R)-3-Hexyl-4-[(R)-2-hydroxytridecyl] -2-oxetanon, Smp. 46-47°; $[\alpha]_D^{20}$ = +12° C ($CHCl_3$, c = 1,5)

aus 3-Hexyl-4-[(R)-2-[(tetrahydro-2H-pyran -2-yl)oxy]tridecyl]-2-oxetanon.

8.H. rac-trans-3-Aethyl-4-(2-hydroxytridecyl) -2-oxetanon, Smp. 35,5-36° C

aus 3-Aethyl-4-[2-[(tetrahydro-2H-pyran -2-yl]oxy]tridecyl]-2-oxetanon,

8.I. trans-3-Methyl-4-[(R)-2-hydroxytridecyl] -2-oxetanon, D.C. Kieselgel, Hexan-Aether 1:3, Rf = 0,49

aus 3-Methyl-4-[(R)-2-[(tetrahydro-2H-pyran -2-yl]oxy]tridecyl]-2-oxetanon.

8.J. rac-trans-3-Allyl-4-[2-hydroxytridecyl] -2-oxetanon, D.C. Kieselgel, Hexan-Aether 1:1, Rf = 0,39

aus 3-Allyl-4-[2-[(tetrahydro-2H-pyran -2-yl]oxy]tridecyl]-2-oxetanon.

8.K. trans-3-Hexyl-4-[(R)-2-hydroxypropyl] -2-oxetanon, D.C. Kieselgel, Hexan-Aether 1:3, Rf = 0,36

aus 3-Hexyl-4-[(R)-2-[(tetrahydro-2H-pyran -2-yl]oxy]propyl]-2-oxetanon.

8.L. rac-trans-3-Hexadecyl-4-(2-hydroxypropyl) -2-oxetanon, Smp. 37-38° C

aus 3-Hexadecyl-4-[2-[(tetrahydro-2H-pyran -2-yl]oxy]propyl]-2-oxetanon.

8.M. rac-trans-3-Hexyl-4-[-2-hydroxy-5-hexenyl] -2-oxetanon(2R,3S,4S:2S,3R,4R)

aus trans-3-Hexyl-4-[-2-[(tetrahydro-2H-pyran -2-yl)oxy]-5-hexenyl]-2-oxetanon.
8.N. trans-3-Decyl-4-[(R)-2-hydroxy-5-hexenyl -2-oxetanon
aus trans-3-Decyl-4-[(R)-2-[(tetrahydro -2H-pyran-2-yl)oxy]hexenyl]-2-oxetanon.
8.O. trans-3-Hexyl-4-((R)-2-hydroxy-5-tridecenyl) -2-oxetanon
aus trans-3-Hexyl-4-[(R)-2-[(tetrahydro -2H-pyran-2-yl]oxy]tridecenyl-2-oxetanon.
8.P. (S)-3-Hexyl-(S)-4[(R) -2-hydroxy-5-hexenyl]-2-oxetanon
aus 3-Hexyl-4-[[(R)-2-[(tetrahydro-2H-pyran -2-yl)oxy]hexenyl]-2-oxetanon.
8.Q. trans-3-Hexyl-4-(2-hydroxytridecyl)-2-oxetanon (Diastereomerengemisch)
aus 3-Hexyl-4-[2-[tetrahydro-2H-pyran -2-yl)oxy]tridecyl]-2-oxetanon.

Beispiel 9

9.A. Herstellung des Produktes

565 mg N-[(Benzyloxy)carbonyl]-L-leucin-(S)-1-[[(2S,3S)-3-äthyl -4-oxo-2-oxetanyl]methyl]octadecylester werden in 12 ml THF gelöst. Man hydriert in Gegenwart von 40 mg 10% Pd/C bei Raumtemperatur. Nach Beendigung der Reaktion wird der Katalysator abfiltriert und eingedampft. Der Rückstand wird in 9 ml THF aufgenommen und 71 $\mu$l Ameisensäureessigsäureanhydrid werden zugetropft. Es wird mit 5 ml Diäthyläther verdünnt und zweimal mit 2% Natriumhydrogencarbonat-Lösung und dann mit Wasser gewaschen. Nach Trocknung über Natriumsulfat wird filtriert und eingedampft. Durch Chromatographie an Kieselgel und Umkristallisation aus n-Pentan erhält man den N-Formyl-(S)-leucin-(S)-1-[[(2S,3S)-3-äthyl-4-oxo-2-oxetanyl]-methyl]octadecylester vom Smp. 60-61 °C.

9.B. Herstellung des Ausgangsmaterials

9.B.a) Wie beschrieben im nachstehenden Absatz 9.B.e) erhält man ein Diastereomerengemisch, das zu 85-90% aus (S,Z)-3-Hydroxy-11-eicosensäure-(R) -2-hydroxy-1,2,2-triphenyläthylester besteht, Smp. 112-114 °C
aus Oleylaldehyd und (R)-$\alpha$-(Hydroxydiphenylmethyl)benzylacetat.
9.B.b) Wie beschrieben im nachstehenden Absatz 9.B.f) erhält man (S,Z)-3-Hydroxy-11-eicosensäuremethylester als farbloses Oel
aus (S,Z)-3-Hydroxy-11-eicosensäure-(R)-2-hydroxy-1,2,2-triphenyläthylester.
9.B.c) Wie im obigen Absatz J)a) beschrieben für die Herstellung der Ester der Formel XV erhält man (S,Z)-3-[(Tetrahydro-2H-pyran-2-yl)oxy]-11-eicosensäure-methylester, welches 10-15% des (R)-Isomeren enthält
aus (S,Z)-3-Hydroxy-11-eicosensäure-methylester.
9.B.d) Wie im obigen Absatz I)a) beschrieben für die Herstellung der Aldehyde der Formel VIII erhält man (S,Z)-3-[(Tetrahydro-2H-pyran-2-yl)oxy] -11-eicosenal, welches 10-15% des entsprechenden (R)-Isomeren enthält
aus (S,Z)-3-[(Tetrahydro-2H-pyran-2-yl)oxy]-11-eicosensäure-methylester.
9.B.e) 7.7 g (R)-$\alpha$-(Hydroxydiphenylmethyl)benzylacetat werden unter Argon in 75 ml THF suspendiert und auf etwa -75 °C gekühlt. Diese Suspension wird tropfenweise mit der doppelten Menge einer Lithium-diisopropylamidlösung versetzt. Man lässt auf 0 °C aufwärmen und rührt 10 Minuten bei dieser Temperatur. Dann wird die Lösung auf -113 bis -117 °C abgekühlt und während des Abkühlens mit 230 ml Diäthyläther versetzt. Zu der Lösung wird eine Lösung von (S,Z)-3-[(Tetrahydro-2H-pyran-2-yl)oxy] -11-eicosenal in 20 ml Diäthyläther zugetropft und noch 30 Minuten gerührt. Es wird tropfenweise mit 20 ml gesättigter Ammoniumchloridlösung versetzt. Man lässt auf Raumtemperatur aufwärmen. Die wässerige Phase wird abgetrennt und die organische Phase wird dreimal mit 80 ml Wasser und einmal mit gesättigter Kochsalzlösung gewaschen. Nach zweimaligem Waschen mit 100 ml gesättigter Ammoniumchloridlösung wird über Natriumsulfat getrocknet, filtriert und eingedampft. Durch mehrmaliges Umkristallisieren aus Methanol erhält man ein Diastereomerengemisch, welches vornehmlich aus (3S,5S,13Z)-3-Hydroxy-5-[(tetrahydro-2H-pyran-2-yl)oxy] -13-docosensäure-(R)-2-hydroxy -1,2,2-triphenyläthylester besteht, Smp. 91-93 °C.
9.B.f) 12,75 g (3S,5S,13Z)-3-Hydroxy-5-[(tetrahydro-2H-pyran-2-yl)oxy] -13-docosensäure-(R)-2-hydroxy-1,2,2-triphenyläthylester werden in 130 ml Methanol suspendiert und mit 17,5 ml 1N methanolische Natriummethylatlösung versetzt. Nachdem die Reaktion beendet ist, wird auf 650 ml gesättigte Ammoniumchloridlösung gegossen und mehrmals mit Diäthyläther extrahiert. Nach Trocknung über Natriumsulfat wird filtriert, eingedampft, der Rückstand wird in 70 ml n-Hexan aufgenommen und 1 Stunde unter

Eisbadkühlung gerührt. Die weissen Kristalle werden abgesaugt und mit n-Hexan gewaschen. Das Filtrat wird eingedampft und an Kieselgel chromatographiert. Man erhält ein Diastereomerengemisch, welches hauptsächlich aus (3S,5S,13Z)-3-Hydroxy-5-[(tetrahydro-2H-pyran-2-yl)oxy] -13-docosensäure-methylester besteht, IR: 3473, 1739, 1076, 1024 $cm^{-1}$.

9.B.g) Wie im obigen Absatz D)a) beschrieben für die Herstellung der Ester der Formel VI erhält man ein Diastereomerengemisch, welches hauptsächlich (2S,3S,5S,13Z)-2-Aethyl-3-hydroxy-5-[(tetrahydro-2H-pyran-2-yl)oxy]-13-docosensäure-methylester enthält, als farbloses Oel

aus (3S,5S,13Z)-3-Hydroxy-5-[(tetrahydro-2H-pyran-2-yl)oxy] -13-docosensäure-methylester und Aethyljodid.

9.B.h) In Analogie zum obigen Beispiel 2 erhält man ein Diastereomerengemisch, das hauptsächlich (2S,3S,5S)-2-Aethyl-3-hydroxy-5-[(tetrahydro-2H-pyran -2-yl)oxy]docosansäure-methylester enthält, IR: 1738, 1199, 1167, 1132, 1115, 1176, 1023 $cm^{-1}$

aus einem Diastereomerengemisch, welches hauptsächlich aus (2S,3S,5S,Z)-2-Aethyl-3-hydroxy-5-[-(tetrahydro-2H-pyran-2-yl)oxy] -13-docosensäure-methylester besteht.

9.B.i) 0,12 g eines Diastereomerengemischs, welches hauptsächlich aus (2S,3S,5S)-2-Aethyl-3-hydroxy-5-[(tetrahydro-2H-pyran-2-yl)oxy]docosansäure-methylester besteht, werden in 2,5 ml 2N methanolischer Kaliumhydroxidlösung bis zum vollständigen Umsatz bei Raumtemperatur gerührt. Die trübe Lösung wird auf 10 ml Wasser gegossen und mit 2N Salzsäure auf pH 2 gestellt. Nach Extraktion mit Diäthyläther wird über Natriumsulfat getrocknet, filtriert und eingedampft. Chromatographie an Kieselgel ergibt ein Diastereomerengemisch, welches hauptsächlich aus (2S,3S,5S)-2-Aethyl-3-hydroxy-5-[-(tetrahydro-2H-pyran-2-yl)oxy]docosansäure besteht, als farbloses Oel, IR: 1709 $cm^{-1}$.

9.B.j) Wie im obigen Absatz A.a) beschrieben für die Herstellung der Aether der Formel IV erhält man (3S,4S)-3-Aethyl-4-[(S)-2-[(tetrahydro-2H-pyran-2-yl)oxy]nonadecyl]-2-oxetanon als Hauptkomponente eines Diastereomerengemisches als farbloses Oel, IR: 1826 $cm^{-1}$

aus einem Diastereomerengemisch, welches hauptsächlich aus (2S,3S,5S)-2-Aethyl-3-hydroxy -5-[-(tetrahydro-2H-pyran-2-yl)oxy]docosansäure besteht.

9.B.k) In Analogie zu Beispiel 7 erhält man (3S,4S)-3-Aethyl-4-[(S)-2-hydroxynonadecyl] -2-oxetanon, Smp. 82-84° C (MeOH)

aus (3S,4S)-3-Aethyl-4-[(S)-2-[(tetrahydro -2H-pyran-2-yl)oxy]nonadecyl]-2-oxetanon.

9.B.l) 796 mg N-[(Benzyloxy)carbonyl]-L-leucin werden in 10 ml Methylenchlorid gelöst, auf 2-3° C abgekühlt und 309 mg Dicyclohexylcarbodiimid zugefügt. Nach 15 Minuten werden die weissen Kristalle abgesaugt und mit Methylenchlorid gewaschen. Das Filtrat wird bei RT im Vakuum eingedampft und der Rückstand in 7 ml N,N-Dimethylformamid (DMF) gelöst. Diese Lösung fügt man zu 574 mg (3S,4S)-3-Aethyl-4-[(S)-2-hydroxynonadecyl] -2-oxetanon und 22 mg 4-Dimethylamino-pyridin in 6 ml DMF. Es wird während 30 Minuten gerührt. Die Lösung wird auf 100 ml Eiswasser gegossen und dreimal mit 20 ml Diäthyläther extrahiert. Die vereinigten organischen Phasen werden über Natriumsulfat getrocknet, filtriert und eingedampft. Nach Chromatographie an Kieselgel erhält man N-[(Benzyloxy)carbonyl]-L-leucin-(S) -1-[[(2S,3S)-3-äthyl-4-oxo-2-oxetanyl]methyl]octadecylester als weisse Kristalle vom Smp. 44-47° C.

Beispiel A

Herstellung von Weichgelatinekapseln folgender Zusammensetzung:

| | Menge pro Kapsel |
|---|---|
| Ein Oxetanon der Formel I oder III<br>NEOBEE M-5 | 50 mg<br>450 µl |

Die Lösung des Wirkstoffes in NEOBEE M-5 wird in Weichgelatinekapseln geeigneter Grösse abgefüllt.

**Patentansprüche**

**Patentansprüche für folgende Vertragsstaaten: BE, CH, DE, FR, GB, IT, LI, LU, NL, SE**

**1.** Oxetanone der Formel

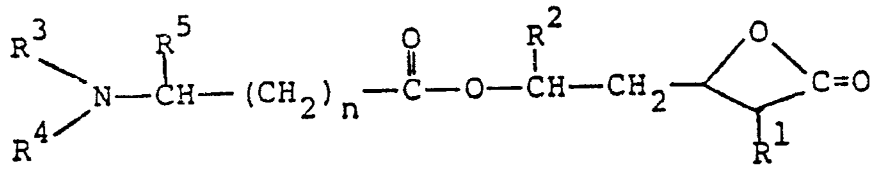

I

worin

R¹ und R² gegebenenfalls durch bis zu 8 Doppel- oder Dreifachbindungen und gegebenenfalls durch ein O- oder S-Atom, das in einer anderen als der α-Stellung zu einem ungesättigten C-Atom vorliegt, unterbrochenes $C_{1-17}$-Alkyl; oder durch 0 bis 3 $C_{1-6}$-Alkyl-(O oder S)$_{1 \text{ oder } 0}$ ringsubstituiertes Phenyl, Benzyl oder $-C_6H_4-X-C_6H_5$,

X Sauerstoff, Schwefel oder $(CH_2)_{0-3}$,

$R^3$ Wasserstoff, $C_{1-3}$-Alkyl oder $C_{1-3}$-Alkanoyl,

$R^4$ Wasserstoff oder $C_{1-3}$-Alkyl, und

$R^5$ Wasserstoff, eine Gruppe Ar oder Ar-$C_{1-3}$-Alkyl oder gegebenenfalls durch Y unterbrochenes und gegebenenfalls durch Z substituiertes $C_{1-7}$-Alkyl sind, oder

$R^4$ mit $R^5$ einen 4- bis 6-gliedrigen gesättigten Ring bildet,

Y Sauerstoff, Schwefel oder eine Gruppe $N(R^6)$, $C(O)N(R^6)$ oder $N(R^6)C(O)$,

Z eine Gruppe -(O oder S)-$R^7$, $-N(R^7,R^8)$,$-C(O)N(R^7,R^8)$ oder $-N(R^7)C(O)R^8$,

n die Zahl 1 oder 0 ist, wobei falls n die Zahl 1 ist, $R^5$ Wasserstoff ist,

Ar durch 0 bis 3 Gruppen $R^9$ oder $OR^9$ substituiertes Phenyl, und

$R^6$ bis $R^9$ Wasserstoff oder $C_{1-3}$-Alkyl sind, wobei, falls $R^3$ Formyl und $R^5$ Isobutyl oder $R^3$ Acetyl und $R^5$ Carbamoylmethyl ist, und gleichzeitig $R^2$ Undecyl oder 2,5-Undecadienyl und $R^1$ n-Hexyl ist, $R^4$ eine andere Bedeutung als Wasserstoff hat,

und Salze dieser Oxetanone mit schwachen Säuren.

**2.** Oxetanone der Formel

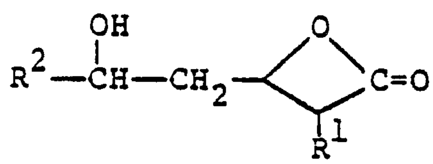

III

worin

R¹ und R² gegebenenfalls durch bis zu 8 Doppel- oder Dreifachbindungen und gegebenenfalls durch ein O- oder S-Atom, das in einer anderen als der α-Stellung zu einem ungesättigten C-Atom vorliegt, unterbrochenes $C_{1-17}$-Alkyl; oder durch bis zu 3 $C_{1-6}$-Alkyl-(O oder S)$_{1 \text{ oder } 0}$ ringsubstituiertes Phenyl, Benzyl oder $-C_6H_4-X-C_6H_5$,und

X Sauerstoff, Schwefel oder $(CH_2)_{0-3}$ sind,

wobei falls $R^1$ n-Hexyl ist, $R^2$ eine andere Bedeutung als Undecyl oder 2,5-Undecadienyl hat.

**3.** Oxetanone nach Anspruch 1 oder 2, worin $R^1$ Methyl, Propyl, Hexyl, Decyl, Hexadecyl, Allyl, Benzyl oder inbesondere Aethyl; $R^2$ Methyl, Undecyl, 3-Butenyl, 3-Undecenyl, 8,11-Heptadecadienyl, Phenoxyphenyl oder insbesondere Heptadecyl; $R^3$ Acetyl oder insbesondere Formyl; $R^4$ Methyl oder insbesondere Wasserstoff, und $R^5$ Wasserstoff, Methyl, 2-Butyl, Benzyl, Methylthioäthyl oder insbesondere i-Butyl ist, oder $R^4$ zusammen mit $R^5$ einen Pyrrolidinylrest bildet.

**4.** N-Formyl-(S)-leucin-(S)-1-[[(2S,3S)-3-äthyl-4-oxo-2-oxetanyl]methyl]octadecylester.

**5.** Ein Oxetanon aus der Gruppe der folgenden

N-Formyl-L-leucin-1-[(trans-3-äthyl-4-oxo -2-oxetanyl)methyl]dodecylester

N-Formyl-L-leucin-1-[(trans-3-allyl-4-oxo -2-oxetanyl)methyl]dodecylester

N-Formyl-(S)-leucin-(S,9Z,12Z)-1-[(2S,3S)-3-äthyl -4-oxo-2-oxetanyl]methyl]-9,12-octadecadienylester

N-Formyl-(S)-leucin-(S,Z)-1-[[(2S,3S)-3-äthyl-4-oxo -2-oxetanyl]methyl]-9-octadecenylester

N-Formyl-(S)-leucin-(R)-α-[[(2S,3S)-3-äthyl-4-oxo-2-oxetanyl]methyl] -p-phenoxybenzylester.

6. Ein Oxetanon gemäss einem der Ansprüche 1 oder 3-5 zur Anwendung als therapeutischer Wirkstoff.

7. Ein Oxetanon gemäss einem der Ansprüche 1 oder 3-5 zur Anwendung als ein die Pankreaslipase hemmender Wirkstoff.

8. Verfahren zur Herstellung eines Oxetanons der Formel I, dadurch gekennzeichnet, dass man
a) eine Säure der Formel

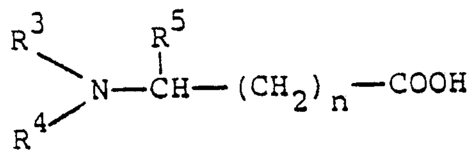

II

oder ein funktionelles Derivat davon mit einem Alkohol der Formel

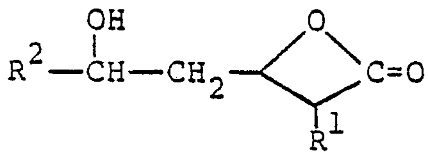

III

worin $R^1$-$R^5$ und n die obige Bedeutung haben, verestert,
b) die Aminoschutzgruppe W in einem Oxetanon der Formel

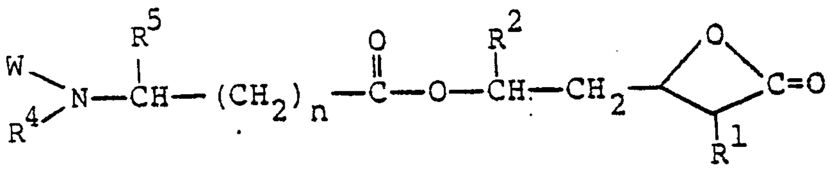

I'

worin $R^1$, $R^2$,$R^4$,$R^5$ und n die obige Bedeutung haben, abspaltet,
c) ungesättigte Reste $R^1$ und $R^2$ gewünschtenfalls katalytisch hydriert,
d) erhaltene Oxetanone der Formel I, worin zumindest eines von $R^3$ und $R^4$ Wasserstoff ist und eine allenfalls in $R^5$ enthaltene Aminogruppe Y oder Z tertiär ist, gewünschtenfalls $C_{1-3}$-alkanoyliert, und
e) erhaltene Oxetanone der Formel I gewünschtenfalls in Form ihrer Salze mit schwachen Säuren isoliert.

9. Verfahren zur Herstellung eines Oxetanons der Formel III, dadurch gekennzeichnet, dass man die Aetherschutzgruppe L in einem Aether der Formel

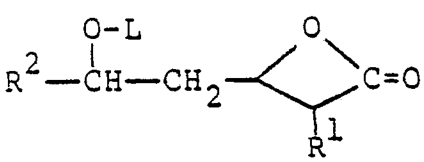

IV

worin L, $R^1$ und $R^2$ die obige Bedeutung haben, abspaltet.

10. Arzneimittel, enthaltend eine Verbindung gemäss einem der Ansprüche 1 oder 3-5 und ein therapeutisch inertes Trägermaterial.

11. Arzneimittel gemäss Anspruch 10, welche die Pankreaslipase hemmen.

12. Verwendung einer Verbindung gemäss einem der Ansprüche 1 oder 3-5 bei der Herstellung von Medikamenten für die Bekämpfung oder Verhütung von Krankheiten.

13. Verwendung einer Verbindung gemäss einem der Ansprüche 1 oder 3-5 bei der Herstellung von Medikamenten für die Bekämpfung oder Verhütung von Obesitas, Hyperlipämien, Atherosklerose und Arteriosklerose.

**Patentansprüche für folgenden Vertragsstaat: AT**

1. Verfahren zur Herstellung von Oxetanonen der Formel

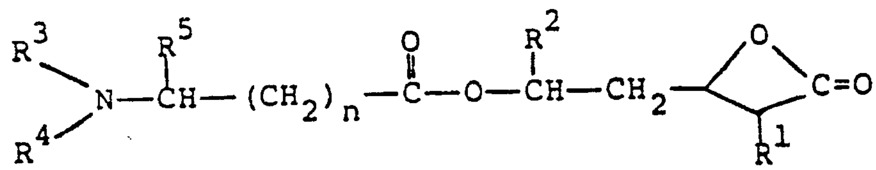

I

worin

| | |
|---|---|
| $R^1$ und $R^2$ | gegebenenfalls durch bis zu 8 Doppel- oder Dreifachbindungen und gegebenenfalls durch ein O- oder S-Atom, das in einer anderen als der α-Stellung zu einem ungesättigten C-Atom vorliegt, unterbrochenes $C_{1-17}$-Alkyl; oder durch 0 bis 3 $C_{1-6}$-Alkyl-(O oder S)$_{1\ \text{oder}\ 0}$ ringsubstituiertes Phenyl, Benzyl oder $-C_6H_4-X-C_6H_5$, |
| X | Sauerstoff, Schwefel oder $(CH_2)_{0-3}$, |
| $R^3$ | Wasserstoff, $C_{1-3}$-Alkyl oder $C_{1-3}$-Alkanoyl, |
| $R^4$ | Wasserstoff oder $C_{1-3}$-Alkyl, und |
| $R^5$ | Wasserstoff, eine Gruppe Ar oder Ar-$C_{1-3}$-Alkyl oder gegebenenfalls durch Y unterbrochenes und gegebenenfalls durch Z substituiertes $C_{1-7}$-Alkyl sind, oder |
| $R^4$ mit $R^5$ | einen 4- bis 6-gliedrigen gesättigten Ring bildet, |
| Y | Sauerstoff, Schwefel oder eine Gruppe $N(R^6)$, $C(O)N(R^6)$ oder $N(R^6)C(O)$, |
| Z | eine Gruppe -(O oder S)-$R^7$, $-N(R^7,R^8)$,$-C(O)N(R^7,R^8)$ oder $-N(R^7)C(O)R^8$, |
| n | die Zahl 1 oder 0 ist, wobei falls n die Zahl 1 ist, $R^5$ Wasserstoff ist, |
| Ar | durch 0 bis 3 Gruppen $R^9$ oder $OR^9$ substituiertes Phenyl, und |

$R^6$ bis $R^9$ Wasserstoff oder $C_{1-3}$-Alkyl sind, wobei, falls $R^3$ Formyl und $R^5$ Isobutyl oder $R^3$ Acetyl und $R^5$ Carbamoylmethyl ist, und gleichzeitig $R^2$ Undecyl oder 2,5-Undecadienyl und $R^1$ n-Hexyl ist, $R^4$ eine andere Bedeutung als Wasserstoff hat, und von Salzen dieser Oxetanone mit schwachen Säuren, dadurch gekennzeichnet, dass man

a) eine Säure der Formel

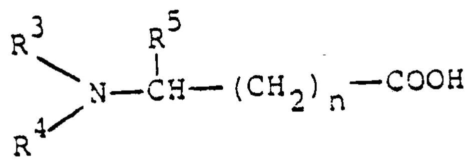

II

oder ein funktionelles Derivat davon mit einem Alkohol der Formel

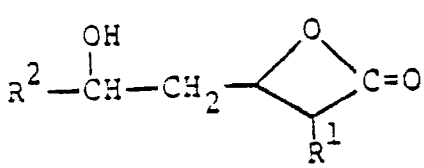

III

worin $R^1$-$R^5$ und n die obige Bedeutung haben, verestert,

b) die Aminoschutzgruppe W in einem Oxetanon der Formel

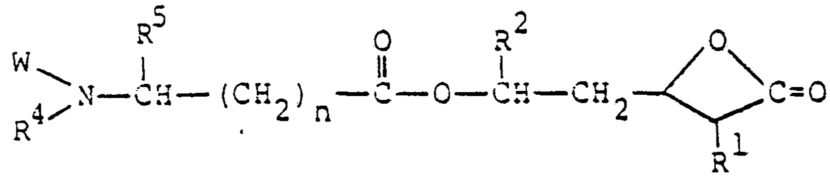

I'

worin $R^1$, $R^2$, $R^4$, $R^5$ und n die obige Bedeutung haben, abspaltet,
c) ungesättigte Reste $R^1$ und $R^2$ gewünschtenfalls katalytisch hydriert,
d) erhaltene Oxetanone der Formel I, worin zumindest eines von $R^3$ und $R^4$ Wasserstoff ist und eine allenfalls in $R^5$ enthaltene Aminogruppe Y oder Z tertiär ist, gewünschtenfalls $C_{1-3}$-alkanoyliert, und
e) erhaltene Oxetanone der Formel I gewünschtenfalls in Form ihrer Salze mit schwachen Säuren isoliert.

2. Verfahren nach Anspruch 1, dadurch gekennzeichnet, dass man die Verfahrensvariante a), b), c) oder e) durchführt und bei Durchführung der Variante a) die freie Säure der Formel II einsetzt.

3. Verfahren zur Herstellung von Oxetanonen der Formel

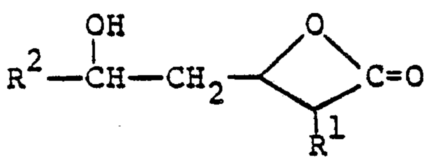

III

worin
$R^1$ und $R^2$ gegebenenfalls durch bis zu 8 Doppel- oder Dreifachbindungen und gegebenenfalls durch ein O- oder S-Atom, das in einer anderen als die $\alpha$-Stellung zu einem ungesättigten C-Atom vorliegt, unterbrochenes $C_{1-17}$-Alkyl; oder durch bis zu 3 $C_{1-6}$-Alkyl-(O oder S)$_{1 \text{ oder } 0}$ ringsubstituiertes Phenyl, Benzyl oder $-C_6H_4-X-C_6H_5$, und
X Sauerstoff, Schwefel oder $(CH_2)_{0-3}$ sind,
wobei falls $R^1$ n-Hexyl ist, $R^2$ eine andere Bedeutung als Undecyl oder 2,5-Undecadienyl hat, dadurch gekennzeichnet, dass man die Aetherschutzgruppe L in einem Aether der Formel

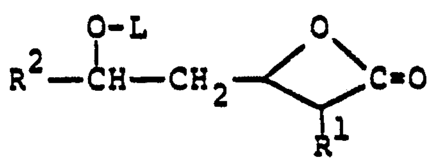

IV

worin L, $R^1$ und $R^2$ die obige Bedeutung haben, abspaltet.

4. Verfahren nach Anspruch 1, 2 oder 3, worin $R^1$ Methyl, Propyl, Hexyl, Decyl, Hexadecyl, Allyl, Benzyl oder inbesondere Aethyl; $R^2$ Methyl, Undecyl, 3-Butenyl, 3-Undecenyl, 8,11-Heptadecadienyl, Phenoxyphenyl oder insbesondere Heptadecyl; $R^3$ Acetyl oder insbesondere Formyl; $R^4$ Methyl oder insbesondere Wasserstoff, und $R^5$ Wasserstoff, Methyl, 2-Butyl, Benzyl, Methylthioäthyl oder insbesondere i-Butyl ist, oder $R^4$ zusammen mit $R^5$ einen Pyrrolidinylrest bildet.

5. Verfahren nach Anspruch 1 oder 2, dadurch gekennzeichnet, dass man N-Formyl-(S)-leucin-(S)-1-[[-(2S,3S)-3-äthyl -4-oxo-2-oxetanyl]methyl]octadecylester herstellt.

6. Verfahren nach Anspruch 1 oder 2, dadurch gekennzeichnet, dass man ein Oxetanon aus der Gruppe der folgenden

N-Formyl-L-leucin-1-[(trans-3-äthyl-4-oxo -2-oxetanyl)methyl]dodecylester
N-Formyl-L-leucin-1-[(trans-3-allyl-4-oxo -2-oxetanyl)methyl]dodecylester
N-Formyl-(S)-leucin-(S,9Z,12Z)-1-[(2S,3S)-3-äthyl -4-oxo-2-oxetanyl]methyl]-9,12-octadecadienylester
N-Formyl-(S)-leucin-(S,Z)-1-[[(2S,3S)-3-äthyl-4-oxo -2-oxetanyl]methyl]-9-octadecenylester
N-Formyl-(S)-leucin-(R)-α-[[(2S,3S)-3-äthyl-4-oxo-2-oxetanyl]methyl] -p-phenoxybenzylester herstellt.

## Claims

### Claims for the following Contracting States: BE, CH, DE, FR, GB, IT, LI, LU, NL, SE

**1.** Oxetanones of the formula

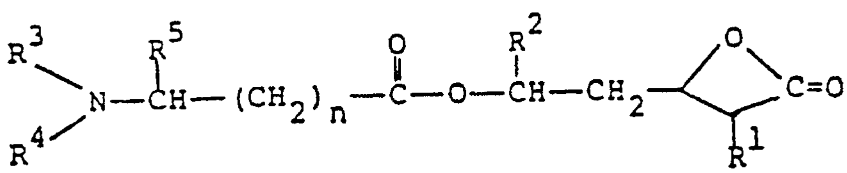

I

wherein

| | |
|---|---|
| $R^1$ and $R^2$ | are $C_{1-17}$-alkyl optionally interrupted by up to 8 double or triple bonds and optionally interrupted by a O or S atom which is present in a position other than the α-position to an unsaturated C-atom; or phenyl, benzyl or $-C_6H_4-X-C_6H_5$ ring-substituted by 0 to 3 $C_{1-6}$-alkyl-(O or S)$_{1 \text{ or } 0}$, |
| X | is oxygen, sulphur or $(CH_2)_{0-3}$, |
| $R^3$ | is hydrogen, $C_{1-3}$-alkyl or $C_{1-3}$-alkanoyl, |
| $R^4$ | is hydrogen or $C_{1-3}$-alkyl, and |
| $R^5$ | is hydrogen, a group Ar or Ar-$C_{1-3}$-alkyl or $C_{1-7}$-alkyl optionally interrupted by Y and optionally substituted by Z, or |
| $R^4$ | forms with $R^5$ a 4- to 6-membered saturated ring, |
| Y | is oxygen, sulphur or a group $N(R^6)$, $C(O)N(R^6)$ or $N(R^6)C(O)$, |
| Z | is a group -(O or S)-$R^7$, $-N(R^7,R^8)$,$-C(O)N(R^7,R^8)$ or $-N(R^7)C(O)R^8$, |
| n | is the number 1 or O, with the proviso that $R^5$ is hydrogen when n is the number 1. |
| Ar | is phenyl substituted by up to 3 groups $R^9$ or $OR^9$, and |
| $R^6$ to $R^9$ | are hydrogen or $C_{1-3}$-alkyl, |

with the proviso that $R^4$ has a significance other than hydrogen when $R^3$ is formyl and $R^5$ is isobutyl or $R^3$ is acetyl and $R^5$ is carbamoylmethyl and simultaneously $R^2$ is undecyl or 2,5-undecadienyl and $R^1$ is n-hexyl, and salts of these oxetanones with weak acids.

**2.** Oxetanones of the formula

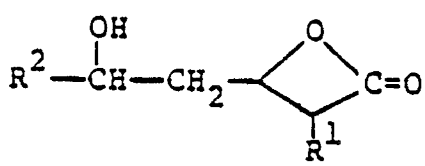

III

wherein

| | |
|---|---|
| $R^1$ and $R^2$ | are $C_{1-17}$-alkyl optionally interrupted by up to 8 double or triple bonds and optionally interrupted by a O or S atom which is present in a position other than the α-position to an unsaturated C atom; or phenyl, benzyl or $-C_6H_4-X-C_6H_5$ ring substituted by up to 3 $C_{1-6}$-alkyl-(O or S)$_{1 \text{ or } 0}$, and |
| X | is oxygen, sulphur or $(CH_2)_{0-3}$, |

With the proviso that $R^2$ has a significance other than undecyl or 2,5-undecadienyl when $R^1$ is n-hexyl.

**3.** Oxetanones according to claim 1 or 2, wherein $R^1$ is methyl, propyl, hexyl, decyl, hexadecyl, allyl, benzyl or especially ethyl; $R^2$ is methyl, undecyl, 3-butenyl, 3-undecenyl, 8,11-heptadecadienyl, phenoxyphenyl or especially heptadecyl: $R^3$ is acetyl or especially formyl; $R^4$ is methyl or especially hydrogen and $R^5$ is hydrogen, methyl, 2-butyl, benzyl, methylthioethyl or especially i-butyl, or $R^4$ and $R^5$ together form a pyrrolidinyl residue.

**4.** N-Formyl-(S)-leucine (S)-1-[[(2S,3S)-3-ethyl-4-oxo-2-oxetanyl]methyl]octadecyl ester.

**5.** An oxetanone from the following group:
N-Formyl-L-leucine 1-[(trans-3-ethyl-4-oxo-2-oxetanyl)methyl]dodecyl ester
N-formyl-L-leucine 1-[(trans-3-allyl-4-oxo-2-oxetanyl)methyl]dodecyl ester
N-formyl-(S)-leucine (S,9Z,12Z)-1-[(2S,3S)-3-ethyl-4-oxo-2-oxetanyl]methyl]-9,12-octadecadienyl ester
N-formyl-(S)-leucine (S,Z)-1-[[(2S,3S)-3-ethyl-4-oxo-2-oxetanyl]methyl]-9-octadecenyl ester
N-formyl-(S)-leucine (R)-α-[[(2S,3S)-3-ethyl-4-oxo-2-oxetanyl]methyl]-p-phenoxybenzyl ester.

**6.** An oxetanone in accordance with any one of claims 1 or 3-5 for use as a therapeutically active substance.

**7.** An oxetanone in accordance with any one of claims 1 or 3-5 for use as an active substance which inhibits pancreas lipase.

**8.** A process for the manufacture of an oxetanone of formula I, characterized by
a) esterifying an acid of the formula

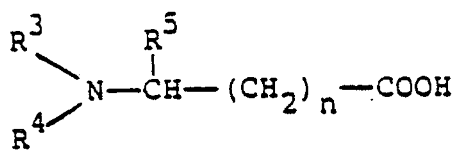

II

or a functional derivative thereof with an alcohol of the formula

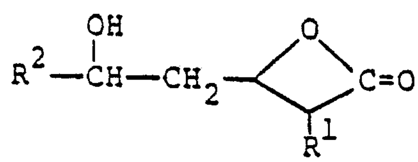

III

wherein $R^1$-$R^5$ and n have the above significance,
b) cleaving off the amino protecting group W in an oxetanone of the formula

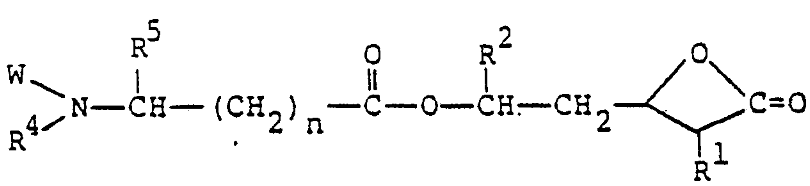

I'

wherein $R^1$, $R^2$, $R^4$, $R^5$ and n have the above significance,
c) if desired, catalytically hydrogenating unsaturated residues $R^1$ and $R^2$,
d) if desired, $C_{1-3}$-alkanoylating oxetanones of formula I obtained in which at least one of $R^3$ and $R^4$ is hydrogen and an amino group Y or Z which may be present in $R^5$ is tertiary, and
e) if desired, isolating oxetanones of formula I obtained in the form of their salts with weak acids.

**9.** A process for the preparation of an oxetanone of formula III, characterized by cleaving off the ether

protecting group L in an ether of the formula

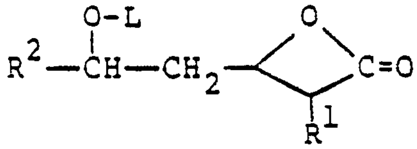

IV

wherein L, $R^1$ and $R^2$ have the above significance.

**10.** A medicament containing a compound in accordance with any one of claims 1 or 3-5 and a therapeutically inert carrier material.

**11.** A medicament in accordance with claim 10, which inhibits pancreas lipase.

**12.** The use of a compound in accordance with any one of claims 1 or 3-5 in the manufacture of medicaments for the control or prevention of illnesses.

**13.** The use of a compound in accordance with any one of claims 1 or 3-5 in the manufacture of medicaments for the control or prevention of obesity, hyperlipaemia, atherosclerosis and arteriosclerosis.

**Claims for the following Contracting State: AT**

**1.** A process for the manufacture of oxetanones of the formula

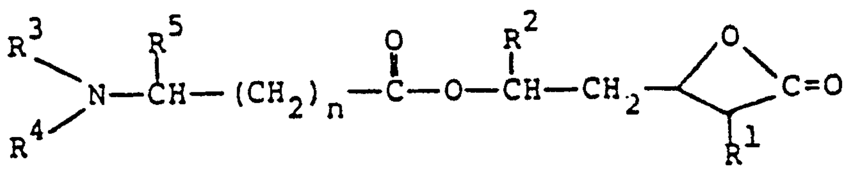

I

wherein

| | |
|---|---|
| $R^1$ and $R^2$ | are $C_{1-17}$-alkyl optionally interrupted by up to 8 double or triple bonds and optionally interrupted by a O or S atom which is present in a position other than the α-position to an unsaturated C-atom; or phenyl, benzyl or $-C_6H_4-X-C_6H_5$ ring-substituted by 0 to 3 $C_{1-6}$-alkyl-(O or S)$_{1 \text{ or } 0}$, |
| X | is oxygen, sulphur or $(CH_2)_{0-3}$, |
| $R^3$ | is hydrogen, $C_{1-3}$-alkyl or $C_{1-3}$-alkanoyl, |
| $R^4$ | is hydrogen or $C_{1-3}$-alkyl, and |
| $R^5$ | is hydrogen, a group Ar or Ar-$C_{1-3}$-alkyl or $C_{1-7}$-alkyl optionally interrupted by Y and optionally substituted by Z, or |
| $R^4$ | forms with $R^5$ a 4- to 6-membered saturated ring, |
| Y | is oxygen, sulphur or a group $N(R^6)$, $C(O)N(R^6)$ or $N(R^6)C(O)$, |
| Z | is a group -(O or S)-$R^7$, $-N(R^7,R^8)$,$-C(O)N(R^7,R^8)$ or $-N(R^7)C(O)R^8$, |
| n | is the number 1 or O, with the proviso that $R^5$ is hydrogen when n is the number 1, |
| Ar | is phenyl substituted by up to 3 groups $R^9$ or $OR^9$, and |
| $R^6$ to $R^9$ | are hydrogen or $C_{1-3}$-alkyl, |

with the proviso that $R^4$ has a significance other than hydrogen when $R^3$ is formyl and $R^5$ is isobutyl or $R^3$ is acetyl and $R^5$ is carbamoylmethyl and simultaneously $R^2$ is undecyl or 2,5-undecadienyl and $R^1$ is n-hexyl, and salts of these oxetanones with weak acids, characterized by

a) esterifying an acid of the formula

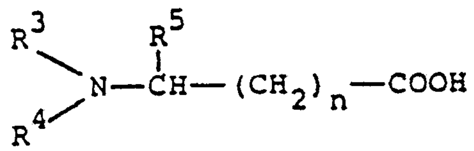

II

or a functional derivative thereof with an alcohol of the formula

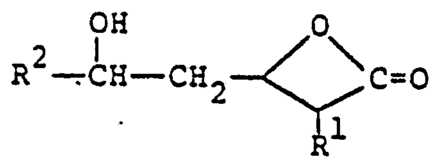

III

wherein $R^1$-$R^5$ and n have the above significance,

b) cleaving off the amino protecting group W in an oxetanone of the formula

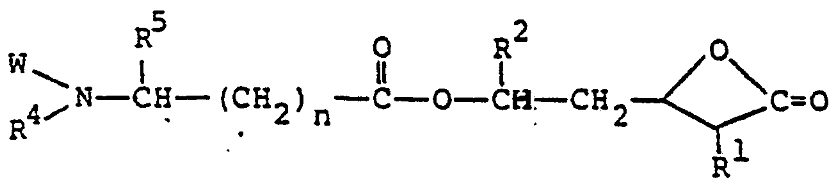

I'

wherein $R^1$, $R^2$, $R^4$, $R^5$ and n have the above significance,

c) if desired, catalytically hydrogenating unsaturated residues $R^1$ and $R^2$,

d) if desired, $C_{1-3}$-alkanoylating oxetanones of formula I obtained in which at least one of $R^3$ and $R^4$ is hydrogen and an amino group Y or Z which may be present in $R^5$ is tertiary, and

e) if desired, isolating oxetanones of formula I obtained in the form of their salts with weak acids.

**2.** A process according to claim 1, characterized in that process variant a), b), c) or e) is carried out and the free acid is used in carrying out variant a).

**3.** A process for the preparation of oxetanones of the formula

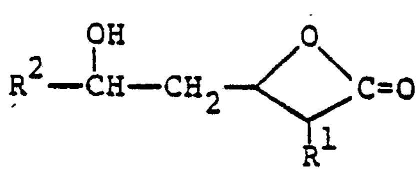

III

wherein

$R^1$ and $R^2$ are $C_{1-17}$-alkyl optionally interrupted by up to 8 double or triple bonds and optionally interrupted by a O or S atom which is present in a position other than the $\alpha$-position to an unsaturated C atom; or phenyl, benzyl or $-C_6H_4-X-C_6H_5$ ring substituted by up to 3 $C_{1-6}$-alkyl-(O or S)$_{1 \text{ or } 0}$, and

X is oxygen, sulphur or $(CH_2)_{0-3}$,

with the proviso that $R^2$ has a significance other than undecyl or 2,5-undecadienyl when $R^1$ is n-hexyl, characterized by cleaving off the ether protecting group L in an ether of the formula

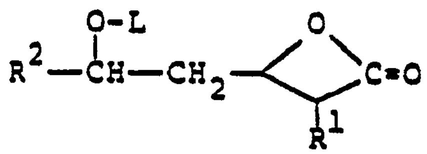

IV

wherein L, $R^1$ and $R^2$ have the above significance.

4. A process according to claim 1, 2 or 3, wherein $R^1$ is methyl, propyl, hexyl, decyl, hexadecyl, allyl, benzyl or especially ethyl; $R^2$ is methyl, undecyl, 3-butenyl, 3-undecenyl, 8,11-heptadecadienyl, phenoxyphenyl or especially heptadecyl; $R^3$ is acetyl or especially formyl; $R^4$ is methyl or especially hydrogen and $R^5$ is hydrogen, methyl, 2-butyl, benzyl, methylthioethyl or especially i-butyl, or $R^4$ and $R^5$ together form a pyrrolidinyl residue.

5. A process according to claim 1 or 2, characterized in that N-formyl-(S)-leucine (S)-1-[[(2S,3S)-3-ethyl-4-oxo-2-oxetanyl]methyl]octadecyl ester is manufactured.

6. A process according to claim 1 or 2, characterized in that an oxetanone from the following group
N-formyl-L-leucine 1-[(trans-3-ethyl-4-oxo-2-oxetanyl)methyl]dodecyl ester
N-formyl-L-leucine 1-[(trans-3-allyl-4-oxo-2-oxetanyl)methyl]dodecyl ester
N-formyl-(S)-leucine (S,9Z,12Z)-1-[(2S,3S)-3-ethyl-4-oxo-2-oxetanyl]methyl]-9,12-octadecadienyl ester
N-formyl-(S)-leucine (S,Z)-1-[[(2S,3S)-3-ethyl-4-oxo-2-oxetanyl]methyl]-9-octadecenyl ester
N-formyl-(S)-leucine (R)-α-[[(2S,3S)-3-ethyl-4-oxo-2-oxetanyl]methyl]-p-phenoxybenzyl ester is manufactured.

**Revendications**
**Revendications pour les Etats contractants suivants: BE, CH, DE, FR, GB, IT, LI, LU, NL, SE**

1. Oxétanones de formule

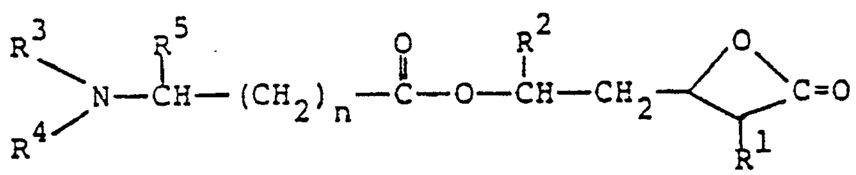

I

où

| | |
|---|---|
| $R^1$ et $R^2$ | sont un alkyle en $C_{1-17}$ interrompu éventuellement par jusqu'à 8 doubles ou triples liaisons et éventuellement par un atome de O ou de S, qui se trouve en une autre position qu'en α par rapport à un atome de C insaturé; ou un phényle, benzyle ou $-C_6H_4-X-C_6H_5$ substitué sur le noyau par 0 à 3 groupes alkyl($C_{1-6}$)-(O ou S)$_{1\ ou\ 0}$, |
| X | est l'oxygène , le soufre ou $(CH_2)_{0-3}$, |
| $R^3$ | est l'hydrogène, un alkyle en $C_{1-3}$ ou un alcanoyle en $C_{1-3}$, |
| $R^4$ | est l'hydrogène ou un alkyle en $C_{1-3}$, et |
| $R^5$ | est l'hydrogène, un groupe Ar ou Ar-alkyle($C_{1-3}$) ou alkyle en $C_{1-7}$ éventuellement interrompu par Y et éventuellement substitué par Z, ou |
| $R^4$ et $R^5$ | forment ensemble un cycle saturé à 4 à 6 chaînons, |
| Y | est l'oxygène, le soufre ou un groupe $N(R^6)$, $C(O)N(R^6)$ ou $N(R^6)C(O)$, |
| Z | est un groupe (O ou S)-$R^7$, -$N(R^7,R^8)$, -$C(O)N(R^7, R^8)$ ou -$N(R^7)C(O)R^8$, |
| n | est le nombre 1 ou 0, $R^5$ étant l'hydrogène quand n est le nombre 1, |
| Ar | est un phényle substitué par 0 à 3 groupes $R^9$ ou $OR^9$, et |

$R^6$ à $R^9$ sont l'hydrogène ou un alkyle en $C_{1-3}$, tandis que, dans le cas où $R^3$ est le formyle et $R^5$ est l'isobutyle ou $R^3$ est l'acétyle et $R^5$ est le carbamoylméthyl, et simultanément $R^2$ est l'undécyle ou le 2,5-undécadiényle et $R^1$ est le n-hexyle, $R^4$ a une autre signification que l'hydrogène,
et les sels de ces oxétanones avec des acides faibles.

2. Oxétanones de formule

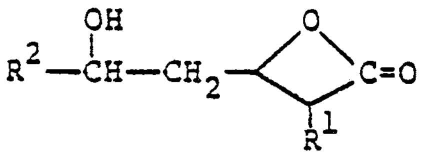

III

où

R¹ et R² sont un alkyle en $C_{1-17}$ interrompu éventuellement par jusqu'à 8 doubles ou triples liaisons et éventuellement par un atome de O ou de S, qui se trouve en une autre position qu'en α par rapport à un atome de C insaturé; ou un phényle, benzyle ou $-C_6H_4-X-C_6H_5$ substitué sur le noyau par 0 à 3 groupes alkyl$(C_{1-6})$-(O ou S)$_{1\ ou\ 0}$,

X est l'oxygène, le soufre ou $(CH_2)_{0-3}$,

tandis que dans le cas où $R^1$ est un n-hexyle $R^2$ a une autre signification qu'undécyle ou 2,5-undécadiényle.

3. Oxétanones selon l'une des revendications 1 ou 2, dans lesquelles $R^1$ est le méthyle, le propyle, l'hexyle, le décyle, l'hexadécyle, l'allyle, le benzyle ou en particulier l'éthyle; $R^2$ est le méthyle, l'undécyle, le 3-butényle, le 3-undécényle, le 8,11-heptadécadiényle, le phénoxyphényle ou en particulier l'heptadécyle; $R^3$ est l'acétyle ou en particulier le formyle; $R^4$ est le méthyle ou en particulier l'hydrogène, et $R^5$ est l'hydrogène, le méthyle, le 2-butyle, le benzyle, le méthylthioéthyle ou en particulier l'iso-butyle, ou bien $R^4$ et $R^5$ forment conjointement un reste pyrrolidinyle.

4. Ester (S)-1-[[(2S,3S)-3-éthyl-4-oxo-2-oxétanyl] méthyl]octadécylique de N-formyl-(S)leucine.

5. Oxétanone choisie parmi les suivantes:
Ester 1-[(trans-3-éthyl-4-oxo-2-oxétanyl)méthyl]dodécylique de N-formyl-L-leucine
Ester 1-[(trans-3-allyl-4-oxo-2-oxétanyl)méthyl]dodécylique de N-formyl-L-leucine
Ester (S,9Z,12Z)-1-[(2S,3S)-3-éthyl-4-oxo-2-oxétanyl]méthyl]-9,12-octadécadiénylique de N-formyl-(S)-leucine
Ester (S,Z)-1-[[(2S,3S)-3-éthyl-4-oxo-2-oxétanyl]méthyl]-9-octadécénylique de N-formyl-(S)-leucine
Ester (R)-α-[[(2S,3S)-3-éthyl-4-oxo-2-oxétanyl]méthyl]-p-phénoxybenzylique de N-formyl-(S)-leucine

6. Oxétanone selon l'une quelconque des revendications 1 ou 3-5 pour l'utilisation comme principe actif thérapeutique.

7. Oxétanone selon l'une quelconque des revendications 1 ou 3-5 pour l'utilisation comme principe actif inhibant la lipase pancréatique.

8. Procédé pour la préparation d'une oxétanone de formule I, caractérisé en ce qu'on
a) estérifie un acide de formule

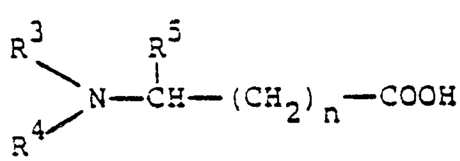

II

ou un dérivé fonctionnel de celui-ci avec un alcool de formule

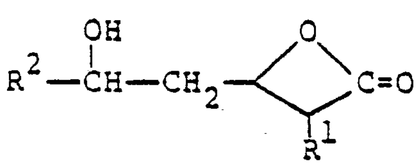

III

où $R^1$-$R^5$ et n ont la signification susdite,

b) élimine le groupe protecteur de l'amino W dans une oxétanone de formule

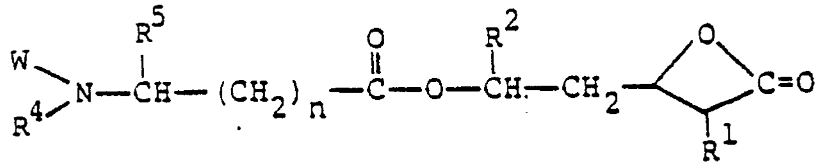

I'

où $R^1$, $R^2$, $R^4$, $R^5$ et n ont la signification susdite,

c) hydrogène catalytiquement si on le souhaite les restes $R^1$ et $R^2$ insaturés,

d) substitue par un alcanoyle en $C_{1-3}$ si on le souhaite les oxétanones obtenues de formule I où au moins un des groupes $R^3$ et $R^4$ est l'hydrogène et un groupe amino Y ou Z éventuellement contenu dans $R^5$ est tertiaire, et

e) isole les oxétanones obtenues de formule I si on le souhaite sous la forme de leurs sels avec des acides faibles.

**9.** Procédé pour la préparation d'une oxétanone de formule III, caractérisé en ce qu'on élimine le groupe protecteur de l'éther L dans un éther de formule

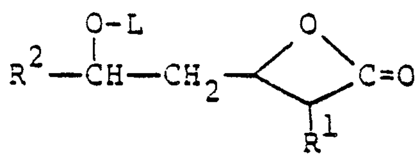

IV

où L, $R^1$ et $R^2$ ont la signification susdite.

**10.** Médicament, contenant un composé selon l'une quelconque des revendications 1 ou 3-5 et une matière support thérapeutiquement inerte.

**11.** Médicament selon la revendication 10, qui inhibe la lipase pancréatique.

**12.** Utilisation d'un composé selon l'une quelconque des revendications 1 ou 3-5 pour la préparation de médicaments pour lutter contre ou prévenir des maladies.

**13.** Utilisation d'un composé selon l'une quelconque des revendications 1 ou 3-5 pour la préparation de médicaments pour lutter contre ou prévenir l'obésité, l'hyperlipémie, l'athérosclérose et l'artériosclérose.

**Revendications pour l'Etat contractant suivant: AT**

**1.** Procédé pour la préparation d'oxétanones de formule

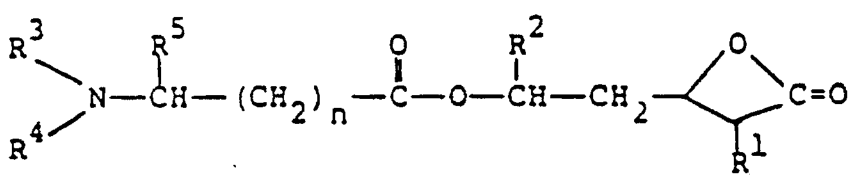

I

où

$R^1$ et $R^2$ sont un alkyle en $C_{1-17}$ interrompu éventuellement par jusqu'à 8 doubles ou triples liaisons et éventuellement par un atome de O ou de S, qui se trouve en une autre position qu'en $\alpha$ par rapport à un atome de C insaturé; ou un phényle, benzyle ou $-C_6H_4-X-C_6H_5$ substitué sur le noyau par 0 à 3 groupes alkyl($C_{1-6}$)-(O ou S)$_{1\ ou\ 0}$,

X est l'oxygène , le soufre ou $(CH_2)_{0-3}$,

$R^3$ est l'hydrogène, un alkyle en $C_{1-3}$ ou un alcanoyle en $C_{1-3}$,
$R^4$ est l'hydrogène ou un alkyle en $C_{1-3}$, et
$R^5$ est l'hydrogène, un groupe Ar ou Ar-alkyle($C_{1-3}$) ou alkyle en $C_{1-7}$ éventuellement interrompu par Y et éventuellement substitué par Z, ou
$R^4$ et $R^5$ forment ensemble un cycle saturé à 4 à 6 chaînons,
Y est l'oxygène, le soufre ou un groupe $N(R^6)$, $C(O)N(R^6)$ ou $N(R^6)C(O)$,
Z est un groupe (O ou S)-$R^7$, -$N(R^7,R^8)$, -$C(O)N(R^7, R^8)$ ou -$N(R^7)C(O)R^8$,
n est le nombre 1 ou 0, $R^5$ étant l'hydrogène quand n est le nombre 1,
Ar est un phényle substitué par 0 à 3 groupes $R^9$ ou $OR^9$, et

$R^6$ à $R^9$ sont l'hydrogène ou un alkyle en $C_{1-3}$, tandis que, dans le cas où $R^3$ est le formyle et $R^5$ est l'isobutyle ou $R^3$ est l'acétyle et $R^5$ est le carbamoylméthyl, et simultanément $R^2$ est l'undécyle ou le 2,5-undécadiényle et $R^1$ est le n-hexyle, $R^4$ a une autre signification que l'hydrogène, et des sels de ces oxétanones avec des acides faibles, caractérisé en ce qu'on

a) estérifie un acide de formule

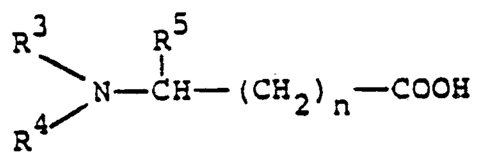
II

ou un dérivé fonctionnel de celui-ci avec un alcool de formule

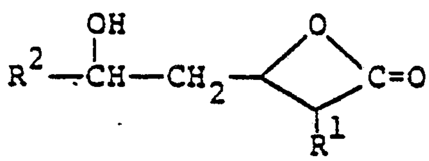
III

ou $R^1$-$R^5$ et n ont la signification susdite,

b) élimine le groupe protecteur de l'amino W dans une oxétanone de formule

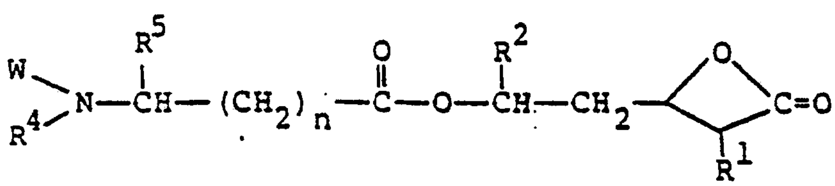
I'

où $R^1$, $R^2$, $R^4$, $R^5$ et n ont la signification susdite,

c) hydrogène catalytiquement si on le souhaite les restes $R^1$ et $R^2$ insaturés,

d) substitue par un alcanoyle en $C_{1-3}$ si on le souhaite les oxétanones obtenues de formule I où au moins un des groupes $R^3$ et $R^4$ est l'hydrogène et un groupe amino Y ou Z éventuellement contenu dans $R^5$ est tertiaire, et

e) isole les oxétanones obtenues de formule I si on le souhaite sous la forme de leurs sels avec des acides faibles.

**2.** Procédé selon la revendication 1, caractérisé en ce qu'on effectue la variante opératoire a), b), c) ou e) et lorsqu'on effectue la variante a) on part de l'acide libre de formule II.

**3.** Procédé pour la préparation d'oxétanones de formule

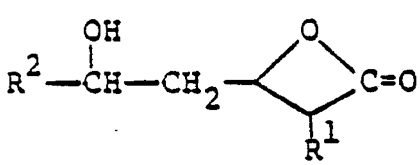
III

où

$R^1$ et $R^2$ sont un alkyle en $C_{1-17}$ interrompu éventuellement par jusqu'à 8 doubles ou triples liaisons et éventuellement par un atome de O ou de S, qui se trouve en une autre position qu'en α par rapport à un atome de C insaturé; ou un phényle, benzyle ou $-C_6H_4-X-C_6H_5$ substitué sur le noyau par 0 à 3 groupes alkyl($C_{1-6}$)-(O ou S)$_{1 \text{ ou } 0}$,

X est l'oxygène, le soufre ou $(CH_2)_{0-3}$,

tandis que dans le cas où $R^1$ est un n-hexyle $R^2$ a une autre signification qu'undécyle ou 2,5-undécadiényle, caractérisé en ce qu'on élimine le groupe protecteur de l'éther L dans un éther de formule

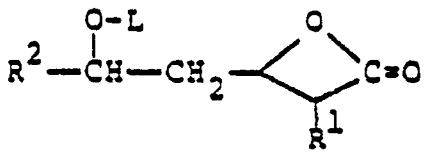

IV

où L, $R^1$ et $R^2$ ont la signification susdite.

4. Procédé selon l'une quelconque des revendications 1, 2 ou 3, dans lequel $R^1$ est le méthyle, le propyle, l'hexyle, le décyle, l'hexadécyle, l'allyle, le benzyle ou en particulier l'éthyle; $R^2$ est le méthyle, l'undécyle, le 3-butényle, le 3-undécényle, le 8,11-heptadécadiényle, le phénoxyphényle ou en particulier l'heptadécyle; $R^3$ est l'acétyle ou en particulier le formyle; $R^4$ est le méthyle ou en particulier l'hydrogène, et $R^5$ est l'hydrogène, le méthyle, le 2-butyle, le benzyle, le méthylthioéthyle ou en particulier l'iso-butyle, ou bien $R^4$ et $R^5$ forment conjointement un reste pyrrolidinyle.

5. Procédé selon l'une des revendications 1 ou 2, caractérisé en ce qu'on prépare l'ester (S)-1-[[(2S,3S)-3-éthyl-4-oxo-2-oxétanyl]méthyl]octadécylique de N-formyl-(S)-leucine.

6. Procédé selon l'une des revendications 1 ou 2, caractérisé en ce qu'on prépare une oxétanone choisie parmi les suivantes:
Ester 1-[(trans-3-éthyl-4-oxo-2-oxétanyl)méthyl]dodécylique de N-formyl-L-leucine
Ester 1-[(trans-3-allyl-4-oxo-2-oxétanyl)méthyl]dodécylique de N-formyl-L-leucine
Ester (S,9Z,12Z)-1-[(2S,3S)-3-éthyl-4-oxo-2-oxétanyl]méthyl]-9,12-octadécadiénylique de N-formyl-(S)-leucine
Ester (S,Z)-1-[[(2S,3S)-3-éthyl-4-oxo-2-oxétanyl]méthyl]-9-octadécénylique de N-formyl-(S)-leucine
Ester (R)-α-[[(2S,3S)-3-éthyl-4-oxo-2-oxétanyl]méthyl]-p-phénoxybenzylique de N-formyl-(S)-leucine.